# EUROPEAN PATENT SPECIFICATION

(11) **EP 2 412 825 B1**
(45) Date of publication and mention of the grant of the patent: **20.09.2017**
(21) Application number: 10756142.5
(22) Date of filing: 24.03.2010
(51) Int. Cl.: C12Q 1/68, A61K 31/713, A61K 35/28

(54) **LEUKEMIA STEM CELL MARKERS**
LEUKÄMIESTAMMZELLENMARKER
MARQUEURS DE CELLULES SOUCHES LEUCÉMIQUES

(30) Priority: 24.03.2009 JP 2009072400
(43) Date of publication of application: 01.02.2012
(73) Proprietor: Riken, Wako-shi, Saitama 351-0198 (JP)
(72) Inventor: ISHIKAWA, Fumihiko, Yokohama-shi Kanagawa 230-0045 (JP); OHARA, Osamu, Yokohama-shi Kanagawa 230-0045 (JP); SAITO, Yoriko, Yokohama-shi Kanagawa 230-0045 (JP); KITAMURA, Hiroshi, Yokohama-shi Kanagawa 230-0045 (JP); HIJIKATA, Atsushi, Yokohama-shi Kanagawa 230-0045 (JP); OZAWA, Hidetoshi, Yokohama-shi Kanagawa 230-0045 (JP); SHULTZ, Leonard D., Bar Harbor Maine 04609 (US)
(74) Representative: J A Kemp
(86) International application number: PCT/JP2010/055131
(87) International publication number: WO 2010/110346

(56) References cited:
- WO-A1-2009/051238
- WO-A1-2009/091547
- WO-A2-2005/045434
- JP-T- 2007 527 238
- JAMIESON C ET AL: "Increased expression of CD47 is a constant marker in mouse and human myeloid leukemias", BLOOD, vol. 106, 1 January 2005 (2005-01-01), page 911A, XP008138745, AMERICAN SOCIETY OF HEMATOLOGY, US ISSN: 0006-4971
- CLARKE MICHAEL F ET AL: "Cancer stem cells--perspectives on current status and future directions: AACR Workshop on cancer stem cells", CANCER RESEARCH, vol. 66, no. 19, 1 October 2006 (2006-10-01), pages 9339-9344, XP002580692, AACR, US PHILADELPHIA, PA ISSN: 1538-7445
- ISHIKAWA FUMIHIKO ET AL: "Chemotherapy-resistant human AML stem cells home to and engraft within the bone-marrow endosteal region", NATURE BIOTECHNOLOGY, vol. 25, no. 11, November 2007 (2007-11), pages 1315-1321, XP002696540, ISSN: 1087-0156
- WILLMAN C L ET AL: "Expression of the c-fgr and hck protein-tyrosine kinases in acute myeloid leukemic blasts is associated with early commitment and differentiation events in the monocytic and granulocytic lineages.", BLOOD, vol. 77, no. 4, 15 February 1991 (1991-02-15), pages 726-734, XP002696541, ISSN: 0006-4971
- FUKAHORI S: "Quantification of WT1 mRNA by competitive NASBA in AML patients.", THE KURUME MEDICAL JOURNAL 2001, vol. 48, no. 2, 2001, pages 129-134, XP002696542, ISSN: 0023-5679
- LINENBERGER M L: "CD33-directed therapy with gemtuzumab ozogamicin in acute myeloid leukemia: progress in understanding cytotoxicity and potential mechanisms of drug resistance", LEUKEMIA, vol. 19, no. 2, 1 February 2005 (2005-02-01), pages 176-182, XP002612906, MACMILLAN PRESS LTD, US ISSN: 0887-6924, DOI: 10.1038/SJ.LEU.2403598 [retrieved on 2004-12-09]
- SAITO YORIKO ET AL: "Identification of therapeutic targets for quiescent, chemotherapy-resistant human leukemia stem cells.", NIH Public Access Author Manuscript, 3 February 2010 (2010-02-03), XP002696543, Retrieved from the Internet: URL:http://www.ncbi.nlm.nih.gov/pmc/articl es/PMC3005290/ [retrieved on 2013-09-05]
- NAOKI HOSEN ET AL.: 'CD96 wa Hakketsubyo Kansaibo Tokuiteki Saibo Hyomen Marker de aru' JAPANESE JOURNAL OF CLINICAL IMMUNOLOGY vol. 30, no. 4, 2007, page 349, XP008154603
- NAOKI HOSEN ET AL.: 'CD96 wa Hito Kyusei Kotsuzuisei Hakketsubyo ni Oite Hakketsubyo Kansaibo to Seijo Zoketsu Kaisaibo o Kubetsu suru Marker de aru' DAI 66 KAI PROCEEDINGS OF THE JAPANESE CANCER ASSOCIATION 20 August 2007, pages 76 - 77, XP008163576
- HOSEN, N. ET AL.: 'CD96 is a leukemic stem cell-specific marker in human acute myeloid leukemia' PROC. NATL. ACAD. SCI. U.S.A. vol. 104, no. 26, 2007, pages 11008 - 11013, XP008109009
- STIREWALT, D., L.. ET AL.: 'Identification of Genes with Abnormal Expression Changes in Acute Myeloid Leukemia' GENES, CHROMOSOMES AND CANCER vol. 47, 2008, pages 8 - 20, XP055015530
- KEIKI KUMANO ET AL.: 'Zoketsuki Shuyo ni Okeru Gan Kansaibo Kenkyu no Shinten: AML Kansaibo Kenkyu no Shinten' HEMATOLOGY & ONCOLOGY vol. 56, no. 6, 28 June 2008, pages 694 - 700, XP008154677
- DORN, D., C. ET AL.: 'The effect of cantharidins on leukemic stem cells' INT. J. CANCER vol. 124, 19 November 2008, pages 2186 - 2199, XP008154594

## Description

### Technical Field

The present invention relates to leukemic stem cell markers and the field of treatment of acute myeloid leukemia.

### Background Art

Acute myeloid leukemia (AML) is the most common/highly frequent (onset rate) adult leukemia, characterized by the clonal expansion of immature myeloblasts initiating from rare leukemic stem cells (LSCs) (non-patent documents 1 - 3). The functional and molecular characteristics of human LSCs are largely undetermined. Although conventional chemotherapeutic agents can temporarily remit AML, recurrence later is the difficult problem that prevents us from helping patients. For the development of an effective therapeutic agent or treatment method, elucidation of the recurrence mechanism by clarifying the leukemia features unknown to date is strongly desired.

A recent study demonstrated that a certain ratio of leukemias and cancers consists of a heterogenous cell fraction and is not configured with a homogenous cell population capable of clonal proliferation. Lapidot and Dick identified such heterogeneity in acute myeloid leukemia (AML) and reported that CD34+CD38- cells are transplanted selectively in CB17-scid and NOD/SCID mice (Non-patent Document 4).

The present inventors have succeeded in the development of an animal model capable of reproducing features of human, rather than mouse, AML, particularly AML of individual patients, rather than a cell line, and permitting long-term assessment (Non-patent Document 5, Patent Application PCT/JP2008/068892). The present inventors further identified using a neonatal NOD/SCID/IL2rg KO mouse model, which is one of the most sensitive human stem cell assays, that CD34+CD38-AML cells meet all criteria for cancer stem cells recommended by the American Association for Cancer Research (Non-patent Document 6). Specifically, CD34+CD38- AML cells self-renew, produce non-stem leukemia cells, and have the exclusive capability of causing AML in living organisms. By repeating primary human AML in NOD/SCID/IL2rg KO mice, the present inventors searched for the mechanism behind the chemotherapy resistance and recurrences, which pose the most important problem in the reality of this disease, and identified the following two essential features of human AML stem cells. First, AML stem cells are present predominantly in the endosteal region of the bone marrow; when human AML transplantation recipient mice were treated with chemotherapeutic agents, the great majority of chemotherapy-resistant AML cells were found in osteoblast niches. Second, AML stem cells (not CD34+CD38+ and CD34-AML cells) are stationary and hence exhibit resistance to cell cycle-dependent chemotherapeutic agents. These histological experiments and cell cycle analyses agree with the clinical evidence that a large number of AML patients achieve remission via chemotherapy induction but eventually experience recurrences. To develop a novel therapeutic strategy designed to exterminate LSCs seems to be an exact step toward overcoming recurrences of AML.

### [Prior art references]

### [non-patent documents]

non-patent document 1: Passegue, E., Jamieson, C.H., Ailles, L.E. & Weissman, I.L. Normal and leukemic hematopoiesis: are leukemias a stem cell disorder or a reacquisition of stem cell characteristics? Proc Natl Acad Sci U S A 100 Suppl 1, 11842-11849 (2003).
non-patent document 2: Hope, K.J., Jin, L. & Dick, J.E. Acute myeloid leukemia originates from a hierarchy of leukemic stem cell classes that differ in self-renewal capacity. Nat Immunol 5, 738-743 (2004).
non-patent document 3: Jordan, C.T. & Guzman, M.L. Mechanisms controlling pathogenesis and survival of leukemic stem cells. Oncogene 23, 7178-7187 (2004).
non-patent document 4: Lapidot, T. et al. A cell initiating human acute myeloid leukaemia after transplantation into SCID mice. Nature 367, 645-648 (1994).
non-patent document 5: Ishikawa, F. et al. Chemotherapy-resistant human AML stem cells home to and engraft within the bone marrow endosteal region. Nature Biotechnol 25:1315-1321 (2007).
non-patent document 6: Clarke, M.F. et al. Cancer stem cellsperspectives on current status and future directions: AACR Workshop on cancer stem cells. Cancer Res 66, 9339-9344 (2006).

### [Summary of the Invention]

### [Problems to Be Solved by the Invention]

A problem to be solved is to find a molecular target that is specific for human leukemic stem cells (LSCs) and provide a therapeutic means that will lead to radical treatment of acute myeloid leukemia (AML) and the like.

### [Means of Solving the Problems]

The present inventors found sets of genes differentially expressed between LSCs and non-stem cells, and proposed the possibility that these genes serve as therapeutic targets for AML (Ishikawa F. et al., Nature Biotechnol 25:1315-1321, 2007 and PCT/JP2008/068892), but were unable to rule out the possibility that the genes are at the same time differentially expressed in normal hematopoietic stem cells (HSCs) as well. Hence, a therapeutic agent and therapeutic method for AML with low prevalence of adverse reactions cannot be realized unless not only a comparison is made between LSCs and non-stem cells, but also a set of genes that are differentially expressed between LSCs and HSCs are identified as targets. The present inventors succeeded in developing a mouse model enabling reproduction of human AML (mice generated by transplanting a fraction containing leukemic stem cells derived from a human AML patient to NOD/SCID/IL2rg^{null} mice), transplanting a small number of bone marrow cells derived from an AML patient, and reconstructing the pathology of AML in the animal model. The present inventors then prepared LSCs derived from an AML patient and those from an AML transplantation recipient mouse, as well as bone marrow samples and cord blood samples (HSCs are contained) derived from healthy donors, conducted a comprehensive analysis, and have developed the present invention.

Accordingly, the present invention provides the following.
[1] A test method for predicting the initial onset or a recurrence of acute myeloid leukemia, comprising
   (1) a step of measuring the expression level of leukemic stem cell marker genes in a biological sample collected from a subject for a transcription product or translation product of the genes as an analyte, and
   (2) a step of comparing the expression levels obtained in the measuring step with a reference value;
      wherein the leukemic stem cell marker genes are selected from:
         (i) 3 - 35 genes, wherein one of the genes is FCGR2A, and the other genes are selected from the group consisting of: cell membrane- or extracellularly-localized genes consisting of ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4;
         (ii) 6 genes consisting of FCGR2A, ITGB2, CD93, CD33, CD3D and TNF;
         (iii) 4 genes consisting of FCGR2A, IL2RA, ITGB2 and CD93; or
         (iv) 8 genes consisting of FCGR2A, AK5, DOK2, LRG1, BIK, IL2RA, WT1 and SUCNR1;
      wherein the reference value for each of the markers is the mean expression value of the marker in CD34+CD38-hematopoietic stem cells for healthy persons or the mean expression value of the marker in CD34+CD38- hematopoietic stem cells for the subject before onset;
      and wherein when the expression of two or more leukemic stem cell marker genes in the subject is significantly higher than the reference value, a possible presence of a leukemic stem cell in the collected biological sample or the subject's body is suggested.
[2] The test method according to [1], wherein the leukemic stem cell marker genes are: (i') 5 - 35 genes, wherein one of the genes is FCGR2A, and the other genes are selected from the group consisting of: cell membrane- or extracellularly-localized genes consisting of ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4.
[3] A production method of a sample containing hematopoietic cells for autologous transplantation or allogeneic transplantation for a patient with acute myeloid leukemia, comprising:
   a) a step of collecting a sample containing hematopoietic cells from the patient or a donor;
   b) a step of bringing the collected sample into contact with antibodies that recognize a translation product of leukemic stem cell marker genes selected from:
      (i) 3 - 35 genes, wherein one of the genes is FCGR2A, and the other genes are selected from the group consisting of:
         cell membrane- or extracellularly-localized genes consisting of ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4;
      (ii) 6 genes consisting of FCGR2A, ITGB2, CD93, CD33, CD3D and TNF;
      (iii) 4 genes consisting of FCGR2A, IL2RA, ITGB2 and CD93; or
      (iv) 8 genes consisting of FCGR2A, AK5, DOK2, LRG1, BIK, IL2RA, WT1 and SUCNR1; and
   c) a step of sorting cells to which the antibodies have bound, and obtaining the sample from which leukemic stem cells have been purged.

### [Effect of the Invention]

The present invention has been developed as a result of succeeding in analyzing the comprehensive expression profiling of leukemic stem cells (LSCs) derived from human primary AML, and identifying LSC-specific targets for separating LSCs from HSCs. Therefore, the leukemic stem cell markers found in the present invention make it possible not only to distinguish between non-stem cells and LSCs, but also to distinguish between normal hematopoietic stem cells (HSCs) and LSCs, which have been thought to be difficult to distinguish from each other. By using a leukemic stem cell marker found in the present invention as a molecular target, a therapeutic agent that acts specifically on LSCs that are the source of onset or recurrence of AML can be provided.

Also, it is possible to specifically remove LSCs from bone marrow cells of a patient or a donor using a cell sorter such as FACS, with a leukemic stem cell marker found in the present invention as an index. This will lead to the effective removal of the true source of onset or recurrences of AML. Therefore, recurrences of AML can be prevented significantly.

Furthermore, the presence or absence of LSCs in a collected biological sample or in a body can be determined with a leukemic stem cell marker found in the present invention as an index, whereby recurrences or the initial onset of acute myeloid leukemia can also be predicted.

### [Brief Description of the Drawings]

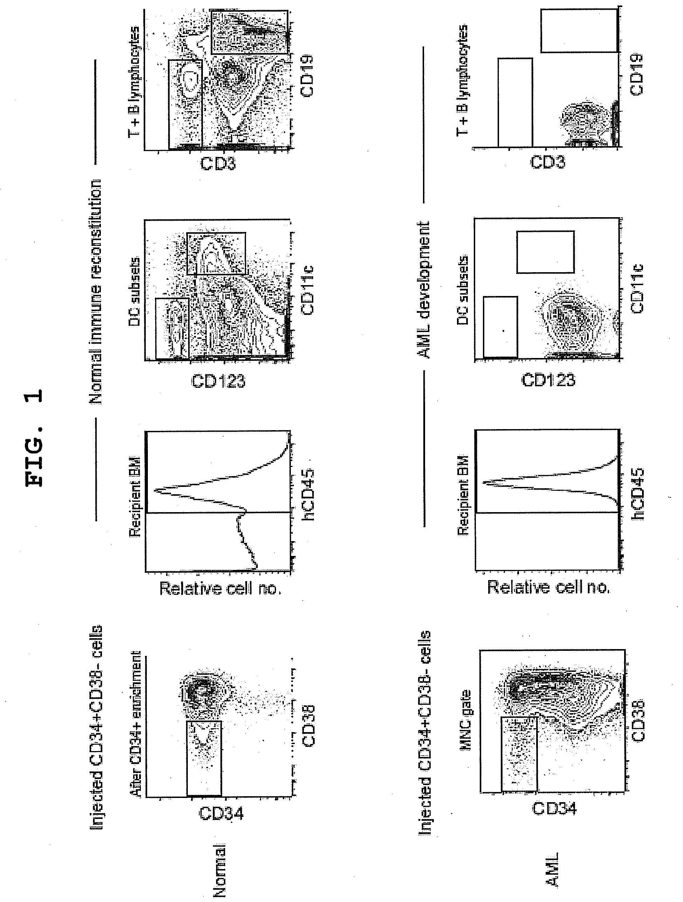

FIG. 1 shows the results of transplantation of normal CD34+CD38- HSCs and AML CD34+CD38- LSCs. (Upper panel) Transplantation of normal CD34+CD38- cells resulted in efficient reconstitution of human CD45+ hematopoietic cells. Because differentiation into normal human immunocytes such as CD11c+ ordinary dendritic cells, CD123-high plasmacytoid dendritic cells, T cells and B cells is observed in human CD45+ cells, it is seen that the CD34+CD38- are hematopoietic stem cells. (Lower panel) When AML CD34+CD38- cells were transplanted, AML developed in recipient mice. Recipient BM was completely occupied by human CD45+ cells, rather than by mouse cells. Because the transplanted human cells did not contain any of normal immunocyte subsets such as dendritic cells, T cells or B cells, the CD34+CD38- cells were shown to contain no normal hematopoietic stem cells and were identified as leukemic stem cells.

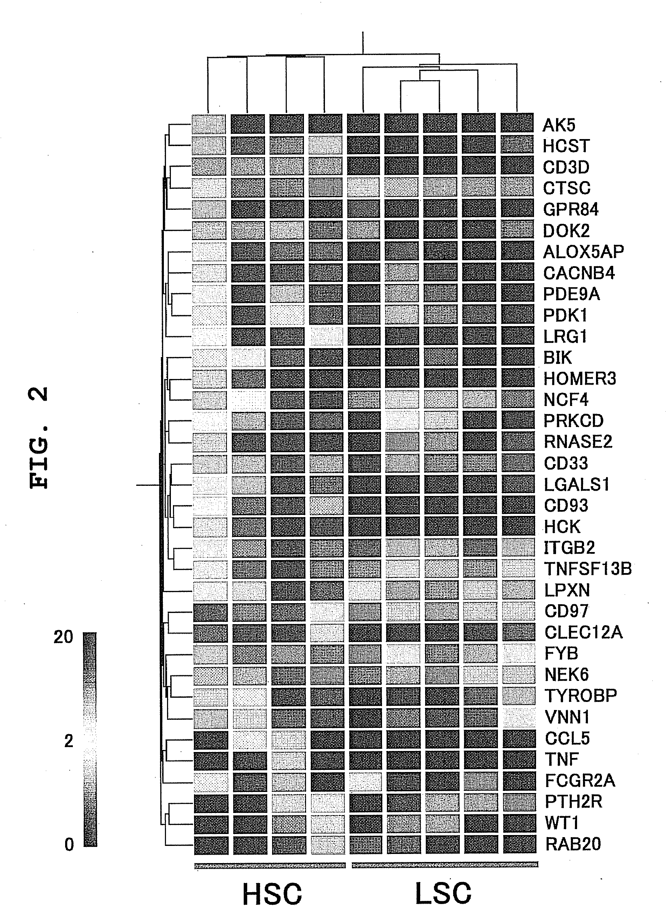

FIG. 2 shows genes expressed in larger amounts in AML CD34+CD38- LSCs than in normal CD34+CD38- HSCs. The heat map includes qPCR data on 35 prominent LSC markers: 1) their functions and localization are suitable for the development of anti-AML drugs, 2) their mRNA contents are significantly (P<0.05) higher in LSCs than in HSCs, 3) the median of their mRNA contents are 5 times or more higher in LSCs than in HSCs, and 4) their mRNA contents are higher in all LSC samples tested than in various HSC samples. In this panel, red, yellow and green indicate high, moderate, and low expression, respectively, as shown by the reference color code in the lower left in this figure. Value 1 indicates the mean for mRNAs in CD34+CD38- HSCs.

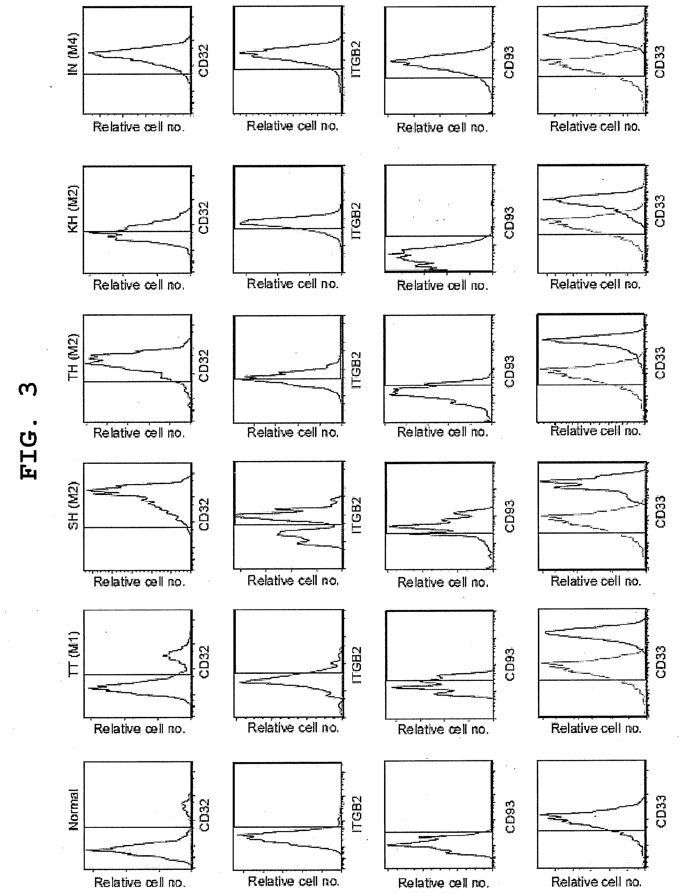

FIG. 3 shows flow cytometry. The expression of LSC-specific molecule candidates (CD32, ITGB2, CD93 and CD33) was analyzed by flow cytometry. Each histogram shows relative expression in LSCs obtained from five AML patients versus that in normal HSCs.

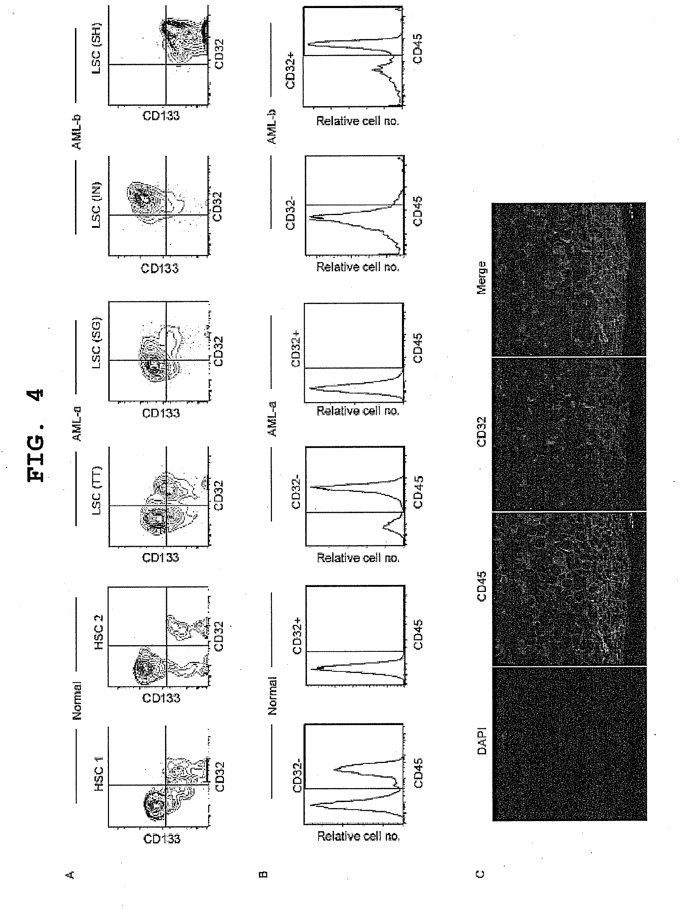

FIG. 4 shows the results of functional assay and histological experiments of CD32. The expression of CD32 and the expression of CD133 were again analyzed by FACS. According to the expression pattern of CD32, AML patients were classified under the categories AML-a and AML-b. Normal HSCs were identified exclusively in the CD32- fraction. Likewise, leukemia induction activity was observed in the CD32- fraction of the AML-a group. In contrast, in AML-b, CD32+ cells exhibited the capability of initiating AML in vivo. In AML-b, CD32+ cells were detected in both the membrane region and central region of the bone marrow.

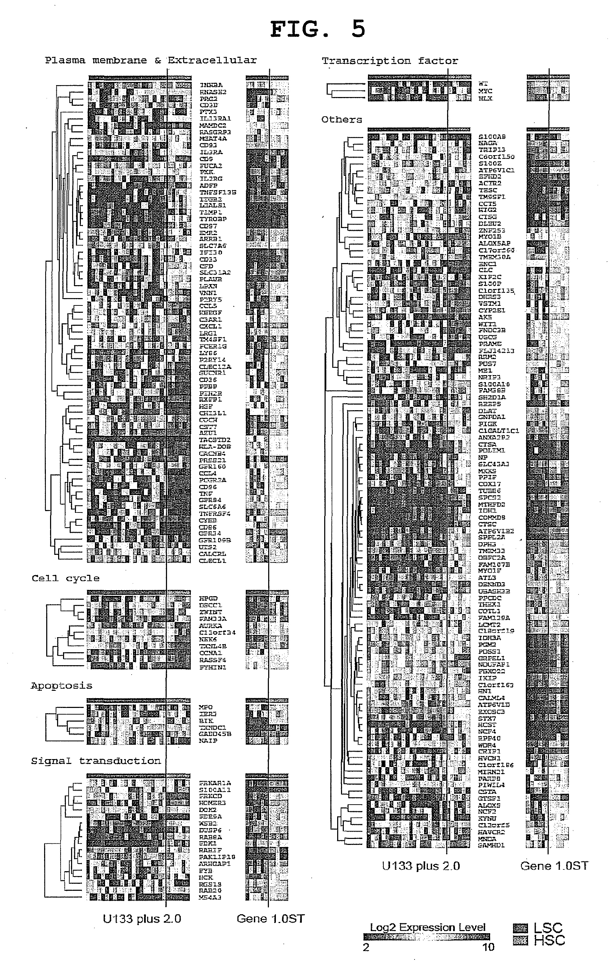

FIG. 5 shows heat map charts of gene candidates whose transcription products are more highly expressed in AML CD34+CD38- LSCs than in normal CD34+CD38- HSCs. 217 genes were classified on the basis of gene ontology under six categories: 1) cell membrane and extracellular, 2) cell cycle, 3) apoptosis, 4) signaling, 5) transcription factors, and 6) others. Gene expression levels on two microarray platforms (U133 plus 2.0 and Gene 1.0ST) are separately shown. In each panel, red, yellow and green indicate high, moderate and low expression, respectively.

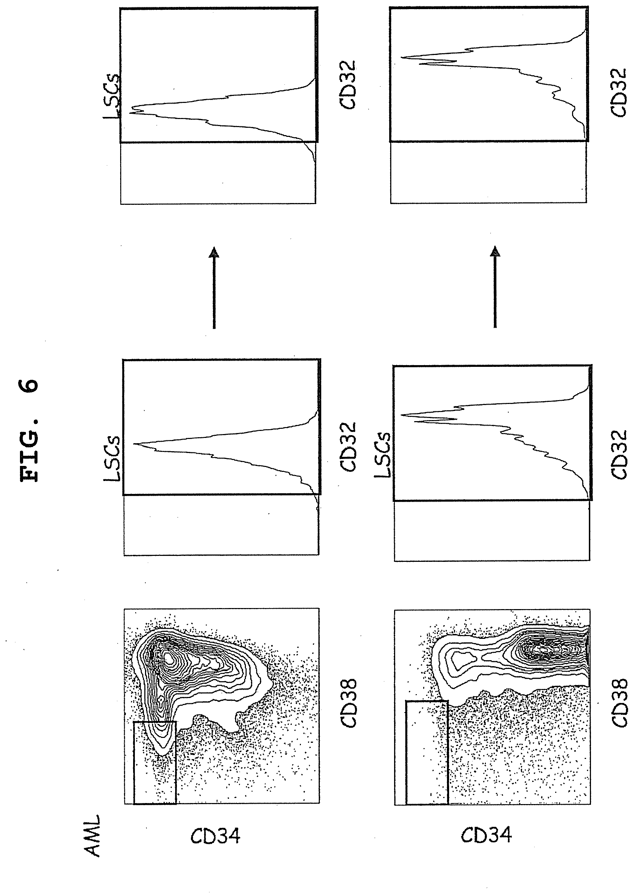

FIG. 6 is a flow cytometric representation showing that the expression of CD32, one of the above-described candidate genes, does not undergo down regulation in AML patients after chemotherapy.

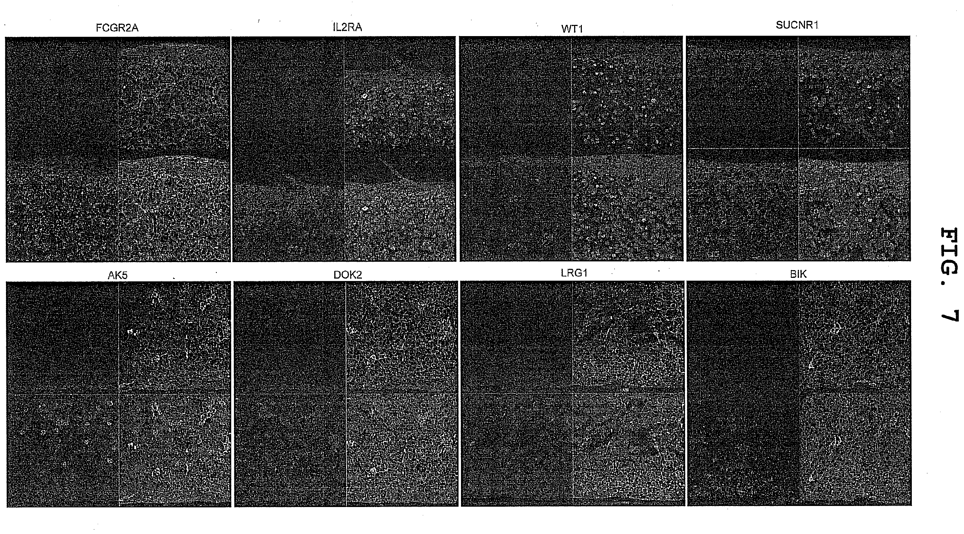

FIG. 7 shows immunofluorescent staining of the expression of various marker genes in leukemic stem cells that are present in bone marrow niches and are in the stationary phase of cell cycle. The results for each gene are shown with a set of four photographs obtained using the DAPI antibody (nuclear staining) for blue staining in the upper left, an antibody against the marker for red staining in the lower left, and an antibody against the cell cycle marker CD34 (in the case of FCGR2A, AK5, DOK2, LRG1, BIK) or the Ki67 antibody (in the case of IL2RA, WT1, SUCNR1) for green staining in the upper right. Shown in the lower right are merged results.

### [Modes for Embodying the Invention]

### (Definitions)

In the present invention, the initial onset of leukemia refers to a state in which leukemia has developed for the first time, or is likely to develop, and a recurrence of leukemia refers to a state in which leukemia has developed again, or is likely to develop, after treatment or remission of initial-onset leukemia. The tissue where leukemia recurs or is likely to recur is not limited to initial-onset tissue, and may be another tissue. Therefore, the concept of recurrence is understood to include infiltration and metastasis.

Treatment of leukemia encompasses all treatments, including administration of anticancer agents, radiotherapy, and bone marrow transplantation.

In the present invention, leukemic stem cells (LSC) may be a CD34+ cell fraction derived from the bone marrow, with preference given to CD34+CD38- cell fraction. The crude substance containing LSC can be recovered from the bone marrow of a test subject or patient by a conventional method, cell fractions containing the LSC can be obtained by flow cytometry and the like using CD34 and CD38 cell surface marker molecules. Note that separation of LSC from HSC is difficult. Furthermore, it is also possible to further sort LSCs with another cell surface marker molecule selected from among leukemic stem cell markers found by the present invention, as an index.

### (Test method)

The present invention provides a test method for predicting the initial onset or a recurrence of acute myeloid leukemia. The test method of the present invention comprises,
(1) a step of measuring the expression level of leukemic stem cell marker genes in a biological sample collected from a subject for a transcription product or translation product of the gene as an analyte, and
(2) a step of comparing the expression levels obtained in the measuring step with the expression level in healthy persons.

### (1) Step of measuring the expression level of leukemic stem cell marker genes in a biological sample collected from a subject for a transcription product or translation product of the gene as an analyte

Leukemic stem cell marker genes are leukemic stem cell-specific markers sorted from a set of genes expressed differentially in the CD34+CD38- cell fraction than in the CD34+CD38+ cell fraction by the present inventors on the basis of their unique viewpoint, and comprise 2 to 218 genes selected from among the following leukemic stem cell marker genes (hereinafter sometimes simply abbreviated as "marker genes" or "markers") (1). The marker genes (1) preferably consist of 3 or more, 5 or more, 10 or more, 15 or more, 20 or more, or 25 or more, genes. The marker genes used in a method of the invention are set out in the claims.

### Marker genes (1):

cell membrane- or extracellularly-localized genes consisting of ADFP, ALOX5AP, AZU1, C3AR1, CACNB4, CALCRL, CCL4, CCL5, CD33, CD36, CD3D, CD86, CD9, CD93, CD96, CD97, CFD, CHI3L1, CLEC12A, CLECL1, COCH, CST7, CXCL1, DOK2, EMR2, FCER1G, FCGR2A, FUCA2, GPR109B, GPR160, GPR34, GPR84, HAVCR2, HBEGF, HCST, HGF, HLA-DOB, HOMER3, IFI30, IL13RA1, IL2RA, IL2RG, IL3RA, INHBA, ITGB2, LGALS1, LRG1, LY86, MAMDC2, MGAT4A, P2RY14, P2RY5, PLAUR, PPBP, PRG2, PRSS21, PTH2R, PTX3, REEP5, RNASE2, RXFP1, SLC31A2, SLC43A3, SLC6A6, SLC7A6, STX7, SUCNR1, TACSTD2, TIMP1, TM4SF1, TM9SF1, TNF, TNFRSF4, TNFSF13B, TYROBP, UTS2 and VNN1;
cell cycle-related genes consisting of AURKA, C13orf34, CCNA1, DSCC1, FAM33A, HPGD, NEK6, PYHIN1, RASSF4, TXNL4B and ZWINT; apoptosis-related genes consisting of MPO, IER3, BIK, TXNDC1, GADD45B and NAIP;
signaling-related genes consisting of AK5, ARHGAP18, ARRB1, DUSP6, FYB, HCK, LPXN, MS4A3, PAK1IP1, PDE9A, PDK1, PRKAR1A, PRKCD, PXK, RAB20, RAB8A, RABIF, RASGRP3, RGS18 and S100A11;
transcription factor genes consisting of WT1, MYC and HLX; and other genes consisting of ACTR2, ALOX5, ANXA2P2, ATL3, ATP6V1B2, ATP6V1C1, ATP6V1D, C12orf5, C17orf60, C18orf19, C1GALT1C1, C1orf135, C1orf163, C1orf186, C6orf150, CALML4, CCT5, CLC, COMMD8, COTL1, COX17, CRIP1, CSTA, CTSA, CTSC, CTSG, CYBB, CYP2E1, DENND3, DHRS3, DLAT, DLEU2, DPH3, EFHD2, ENC1, EXOSC3, FAM107B, FAM129A, FAM38B, FBXO22, FLJ14213, FNDC3B, GNPDA1, GRPEL1, GTSF1, HIG2, HN1, HVCN1, IDH1, IDH3A, IKIP, KIF2C, KYNU, LCMT2, ME1, MIRN21, MKKS, MNDA, MTHFD2, MYO1B, MYO1F, NAGA, NCF2, NCF4, NDUFAF1, NP, NRIP3, OBFC2A, PARP8, PDLIM1, PDSS1, PGM2, PIGK, PIWIL4, PPCDC, PPIF, PRAME, PUS7, RPP40, RRM2, S100A16, S100A8, S100P, S100Z, SAMHD1, SH2D1A, SPCS2, SPPL2A, TESC, THEX1, TMEM30A, TMEM33, TRIP13, TUBB6, UBASH3B, UGCG, VSTM1, WDR4, WIT1, WSB2 and ZNF253.

The individual genes that constitute the aforementioned leukemic stem cell marker genes are publicly known, and the base sequences and amino acid sequences thereof are also known. For the marker genes except IL2RA, symbol names, gene IDs, location chromosomes, characteristics and the like are shown in Table 1. IL2RA, also called CD25 has the gene ID 3559, is located on chromosome 10, and encodes interleukin 2 receptor alpha. The IL2RA protein is a transmembranous receptor localized on the cell membrane.

**[Table 1-1]**

| symbol | geneID | probeID U133 | probeID GeneST | Fold Change (AML/Health y, U133) | Fold Change (AML/Health y, GeneST) | chrom osome | explanation | function | location | process |
|---|---|---|---|---|---|---|---|---|---|---|
| ACTR2 | 10097 | 1558015_s_at | 8042337 | 4.2638 | 1.8188 | 2 | ARP2 actin-related protein 2 homolog (yeast) | | cytoplasm | |
| ADFP | 123 | 209122_at | 8160297 | 3.2797 | 2.3209 | 9 | adipose differentiation-related protein | | cell membrane | |
| AK5 | 26289 | 222862_s_at | 7902452 | 3.1895 | 16.5796 | 1 | adenylate kinase 5 | signaling molecule | cytoplasm | |
| ALOX5 | 240 | 204446_s_at | 7927215 | 18.4004 | 2.6463 | 10 | arachidonate 5-lipoxygenase | | cytoplasm | |
| ALOX5AP | 241 | 204174_at | 7968344 | 2.8061 | 9.5599 | 13 | arachidonate 5-lipoxygenase-activating protein | | cell membrane | |
| ANXA2P2 | 304 | 208816_x_at | 8154836 | 4.5495 | 4.8032 | 9 | annexin A2 pseudogene 2 | | unknown | |
| ARHGAP18 | 93663 | 225171_at | 8129458 | 4.3548 | 2.5895 | 6 | Rho GTPase activating protein 18 | signaling molecule | unknown | |
| ARRB1 | 408 | 222912_at | 7950473 | 3.8043 | 1.9404 | 11 | arrestin, beta 1 | signaling molecule | cytoplasm | |
| ATL3 | 25923 | 223452_s_at | 7948997 | 3.6187 | 1.8605 | 11 | atlastin 3 | | unknown | |
| ATP6V1B2 | 526 | 201089_at | 8144931 | 4.0733 | 2.0284 | 8 | ATPase, H+ transporting, lysosomal 56/58kDa, V1 subunit B2 | | cytoplasm | |
| ATP6V1C1 | 528 | 202872_at | 8147724 | 3.2006 | 2.3894 | 8 | ATPase, H+ transporting, lysosomal 42kDa, V1 subunit C1 | | cytoplasm | |
| ATP6V1D | 51382 | 208899_x_at | 7979698 | 4.6313 | 2.2873 | 14 | ATPase, H+ transporting, lysosomal 34kDa, V1 subunit D | | cytoplasm | |
| AURKA | 6790 | 208079_s_at | 8067167 | 20444 | 2.4771 | 20 | aurora kinase A | signaling molecule | nucleus | cell cycle |
| AZU1 | 566 | 214575_s_at | 8024038 | 3.3641 | 4.0737 | 19 | azurocidin 1 | | extracellular space | |
| BIK | 638 | 205780_at | 8073605 | 5.3934 | 8.8991 | 22 | BCL2-interacting killer (apoptosis-inducing) | | cytoplasm | apoptosis |
| C12orf5 | 57103 | 219099_at | 7953211 | 6.1678 | 4.4117 | 12 | chromosome 12 open reading frame 5 | | unknown | |
| C13orf34 | 79866 | 219544_at | 7969374 | 3.7631 | 2.3038 | 13 | chromosome 13 open reading frame 34 | | unknown | cell cycle |
| C17orf60 | 284021 | 217513_at | 8009243 | 3.2027 | 3.7201 | 17 | chromosome 17 open reading frame 60 | | unknown | |
| C18orf19 | 125228 | 235022_at | 8022404 | 2.828 | 1.8669 | 18 | chromosome 18 open reading frame 19 | | unknown | |
| C1GALT1C1 | 29071 | 219283_at | 8174820 | 4.6168 | 2.2689 | X | C1GALT1-specific chaperone 1 | | unknown | |
| C1orf135 | 79000 | 220011_at | 7913852 | 2.8168 | 2.9438 | 1 | chromosome 1 open reading frame 135 | | unknown | |
| C1orf163 | 65260 | 219420_s_at | 7916219 | 2.9798 | 2.4301 | 1 | chromosome 1 open reading frame 163 | | unknown | |
| C1orf186 | 440712 | 230381_at | 7923875 | 10.2556 | 2.3674 | 1 | chromosome 1 open reading frame 186 | | unknown | |
| C3AR1 | 719 | 209906_at | 7960874 | 8.2753 | 4.4025 | 12 | complement component 3a receptor 1 | transmembranous receptor | cell membrane | |
| C6orf150 | 115004 | 1559051_s_at | 8127534 | 2.9862 | 3.4927 | 6 | chromosome 6 open reading frame 150 | | unknown | |
| CACNB4 | 785 | 207693_at | 8055872 | 2.8303 | 2.6543 | 2 | calcium channel, voltage-dependent, beta 4 subunit | | cell membrane | |
| CALCRL | 10203 | 208331_at | 8067578 | 2.5601 | 2.2268 | 2 | calcitonin receptor-like | transmembranous receptor | cell membrane | |

**[Table 1-2]**

| | | | | | | | | | | |
|---|---|---|---|---|---|---|---|---|---|---|
| CALML4 | 91860 | 221879_at | 7989968 | 2.5411 | 2.2694 | 15 | calmodulin-like 4 | | unknown | |
| CCL4 | 6351 | 204103_at | 8006602 | 12.0201 | 21087 | 17 | chemokine (C-C motif) ligand 4 | cytokine and growth factor | extracellular space | |
| CCL5 | 6352 | 1405_i_at | 8014316 | 13.0433 | 10.0074 | 17 | chemokine (C-C motif) ligand 5 | cytokine and growth factor | extracellular space | immunity, cell adhesion |
| CCNA1 | 8900 | 205899_at | 7968637 | 3.325 | 3.9705 | 13 | cyclin A1 | | nucleus | cell cycle |
| CCT5 | 22948 | 229068_at | 8104449 | 2.828 | 2.0069 | 5 | chaperonin containing TCP1, subunit 5 (epsilon) | | cytoplasm | |
| CD33 | 945 | 206120_at | 8030804 | 3.4258 | 4.0167 | 19 | CD33 molecule | signaling molecule | cell membrane | cell adhesion |
| CD36 | 948 | 228766_at | 8133876 | 6.3287 | 2.1815 | 7 | CD36 molecule (thrombospondin receptor) | | cell membrane | |
| CD3D | 915 | 213539_at | 7952056 | 6.673 | 11.2019 | 11 | CD3d molecule, delta (CD3-TCR complex) | transmembranous receptor | cell membrane | |
| CD86 | 942 | 210895_s_at | 8082035 | 3.6193 | 4.1863 | 3 | CD86 molecule | tranemembranous receptor | cell membrane | |
| CD9 | 928 | 201005_at | 7953291 | 28.019 | 1.7512 | 12 | CD9 molecule | | cell membrane | |
| CD93 | 22918 | 202878_s_at | 8065359 | 13.7302 | 1.9706 | 20 | CD93 molecule | | cell membrane | cell adhesion |
| CD96 | 10225 | 206761_at | 8081564 | 4.247 | 4.9945 | 3 | CD96 molecule | | cell membrane | |
| CD97 | 976 | 202910_s_at | 8026300 | 7.6085 | 20902 | 19 | CD97 molecule | transmembranous receptor | cell membrane | immunity, cell adhesion |
| CFD | 1675 | 205382_s_at | 8024062 | 7.7147 | 3.955 | 19 | complement factor D (adipsin) | | extraoellular space | |
| CHI3L1 | 1116 | 209395_at | 7923547 | 3.3299 | 2.3625 | 1 | chltinase 3-like 1 (cartilage glycoprotein-39) | | extracellular space | |
| CLC | 1178 | 206207_at | 8038755 | 4.9719 | 20.5111 | 19 | Charcot-Leyden crystal protein | | cytoplasm | |
| CLEC12A | 160364 | 1552398_s_at | 7953901 | 14.7668 | 10.4421 | 12 | C-type lectin domain family 12, member A | | cell membrane | |
| CLECL1 | 160365 | 244413_at | 7961069 | 3.2716 | 9.2279 | 12 | C-type lectin-like 1 | | cell membrane | |
| COCH | 1690 | 205229_s_at | 7973797 | 2.6217 | 2.5193 | 14 | coagulation factor C homolog, cochlin (Limulus polyphemus) | | extracellular space | |
| COMMD8 | 54951 | 218351_at | 8100145 | 4.698 | 2.2621 | 4 | COMM domain containing 8 | | unknown | |
| COTL1 | 23406 | 224583_at | 8003171 | 4.2253 | 1.9761 | 16 | coactosin-like 1 (Dictyostelium) | | cytoplasm | |
| COX17 | 10063 | 203880_at | 7968972 | 2.9867 | 20975 | 3 | COX17 cytochrome c oxidase assembly homolog (S. cerevisiae) | | cytoplasm | |
| CRIP1 | 1396 | 205081_at | 7977409 | 10.2268 | 1.9145 | 14 | cysteine-rich protein 1 (intestinal) | | cytoplasm | |
| CST7 | 8530 | 210140_at | 8061416 | 4.5721 | 4.3944 | 20 | cystatin F (leukocystatin) | | extracellular space | |
| CSTA | 1476 | 204971_at | 8082058 | 15.2724 | 8.2554 | 3 | cystatin A (stefin A) | | cytoplasm | |
| CTSA | 5476 | 200661_at | 8063078 | 7.4704 | 2.0204 | 20 | cathepsin A | | cytoplasm | |
| CTSC | 1075 | 201487_at | 7950906 | 4.4802 | 3.1109 | 11 | cathepsin C | | cytoplasm | immunity |
| CTSG | 1511 | 205653_at | 7978351 | 4.5766 | 5.8038 | 14 | cathepsin G | | cytoplasm | Immunity |
| CXCL1 | 2919 | 204470_at | 8095697 | 11.0827 | 2.1273 | 4 | chemokine (C-X-C motif) ligand 1 (melanoma growth stimulating | cytokine and growth factor | extracellular space | |

**[Table 1-3]**

| | | | | | | | | | | |
|---|---|---|---|---|---|---|---|---|---|---|
| CYBB | 1536 | 203923_s_at | 8166730 | 3.9235 | 4.1921 | x | cytochrome b-245, beta polypeptide | | cytoplasm | immunity |
| CYP2E1 | 1571 | 209975_at | 7931643 | 2.58 | 2.2439 | 10 | cytochrome P450, family 2. subfamily E. polypeptide 1 | | cytoplasm | |
| DENND3 | 22898 | 212975_at | 8148476 | 2.9132 | 2.0985 | 8 | DENN/MADD domain containing 3 | | unknown | |
| DHRS3 | 9249 | 202481_at | 7912537 | 3.7799 | 2.499 | 1 | dehydrogenase/reductase (SDR family) member 3 | | cytoplasm | |
| DLAT | 1737 | 212568_s_at | 7943827 | 5.313 | 2.2002 | 11 | dihydrolipoamide S-acetyltransferase | | cytoplasm | |
| DLEU2 | 8847 | 1556821_x_at | 7971653 | 2.876 | 3.9304 | 13 | deleted in lymphocytic leukemia 2 (non-protein coding) | | unknown | |
| DOK2 | 9046 | 214054_at | 8149638 | 5.6934 | 3.0391 | 8 | docking protein 2, 56kDa | signaling molecule | cell membrane | |
| DPH3 | 285381 | 225200_at | 8085660 | 2.875 | 2.0278 | 3 | DPH3, KTI11 homolog (S. cerevisiae) | | cytoplasm | |
| DSCC1 | 79075 | 219000_s_at | 8152582 | 2.5694 | 2.6348 | 8 | defective in sister chromatid cohesion 1 homolog (S. cerevisiae) | | nucleus | cell cycle |
| DUSP6 | 1848 | 208893_s_at | 7965335 | 3.9521 | 2.0696 | 12 | dual specificity phosphatase 6 | signaling molecule | cytoplasm | |
| EFHD2 | 79180 | 222483_at | 7898161 | 3.0525 | 2.0378 | 1 | EF-hand domain family, member D2 | | unknown | |
| EMR2 | 30817 | 207610_s_at | 8034873 | 10.5352 | 2.0458 | 19 | egf-like module containing, mucin-like, hormone receptor-like 2 | | cell membrane | |
| ENC1 | 8507 | 201341_at | 8112615 | 6.3235 | 1.8298 | 5 | ectodermal-neural cortex (with BTB-like domain) | | nucleus | |
| EXOSC3 | 51010 | 227916_x_at | 8161242 | 3.051 | 2.019 | 9 | exosome component 3 | | nucleus | |
| FAM107B | 83641 | 223058_at | 7932160 | 11.3343 | 2.1322 | 10 | family with sequence similarity 107, member B | | nucleus | |
| FAM129A | 116496 | 217966_s_at | 7922846 | 7.1413 | 1.714 | | family with sequence similarity 129, member A | | cytoplasm | |
| FAM33A | 348235 | 225684_at | 8017133 | 2.6048 | 2.0228 | 17 | family with sequence similarity 33, member A | | nucleus | cell cycle |
| FAM38B | 63895 | 219602_s_at | 8022283 | 2.7684 | 2.4385 | 18 | family with sequence similarity 38, member B | | unknown | |
| FBXO22 | 26263 | 226734_at | 7985053 | 3.3569 | 1.8734 | 15 | F-box protein 22 | | unknown | |
| FCER1G | 2207 | 204232_at | 7906720 | 5.172 | 4.5138 | 1 | Fo fragment of IgE, high affinity I, receptor for, gamma polypeptide | transmembranous receptor | cell membrane | Immunity, apoptosis |
| FCGR2A | 2212 | 203561_at | 7906757 | 3.6163 | 4.5895 | 1 | Fc fragment of IgG, low affinity IIa, receptor (GD32) | transmembranous receptor | cell membrane | |
| FLJ14213 | 79899 | 233379_at | 7939383 | 3.2662 | 1.8592 | 11 | protor-2 | | unknown | |
| FNDC3B | 64778 | 222692_s_at | 8083901 | 4.0438 | 1.815 | 3 | fibronectin type III domain containing 3B | | unknown | |
| FUCA2 | 2519 | 223120_at | 8129974 | 2.7625 | 2.214 | 6 | fucosidase, alpha-L-2. plasma | | extracellular space | |
| FYB | 2533 | 227266_s_at | 8111739 | 5.75 | 3.4782 | 5 | FYN binding protein (FYB-120/130) | signaling molecule | nucleus | immunity |
| GADD45B | 4616 | 209305_s_at | 8024485 | 8.2835 | 1.8588 | 19 | growth arrest and DNA-damage-inducible, beta | | cytoplasm | apoptosis |
| GNPDA1 | 10007 | 202382_s_at | 8114787 | 4.3678 | 1.8908 | 5 | glucosamine-6-phosphate deaminase 1 | | cytoplasm | |
| GPR109B | 8843 | 205220_at | 7967322 | 25.4362 | 5.9615 | 12 | G protein-coupled receptor 109B | transmembranous receptor | cell membrane | |
| GPR160 | 26996 | 223423_at | 8083839 | 24534 | 2.4379 | 3 | G protein-coupled receptor 160 | transmembranous receptor | cell membrane | |
| GPR34 | 2857 | 223620_at | 8166906 | 3.7631 | 2.7359 | X | G protein-coupled receptor 34 | transmembranous receptor | cell membrane | |

**[Table 1-4]**

| | | | | | | | | | | |
|---|---|---|---|---|---|---|---|---|---|---|
| GPR84 | 53831 | 223767_at | 7963770 | 3.5766 | 2.6827 | 12 | G protein-coupled receptor 84 | transmembranous receptor | cell membrane | |
| GRPEL1 | 80273 | 212432_at | 8099246 | 8.8722 | 2.1952 | 4 | GrpE-like 1, mitochondrial (E. coli) | | cytoplasm | |
| GTSF1 | 121355 | 227711_at | 7963817 | 8.6795 | 5.142 | 12 | gametocyte specific factor 1 | | cytoplasm | |
| HAVCR2 | 84868 | 235458_at | 8115464 | 3.8093 | 2.0482 | 5 | hepatitis A virus cellular receptor 2 | transmembranous receptor | cell membrane | |
| HBEGF | 1839 | 203821_at | 8114572 | 19.1502 | 3.4502 | 5 | heparin-binding EGF-like growth factor | cytokine and growth factor | extracellular space | |
| HCK | 3055 | 208018_s_at | 8061668 | 17.6625 | 4.7152 | 20 | hemopoletic cell kinase | signaling molecule | cytoplasm | |
| HOST | 10870 | 223640_at | 8028104 | 4.0478 | 2.9073 | 19 | hematopoletic cell signal transducer | | cell membrane | |
| HGF | 3082 | 210997_at | 8140556 | 4.5623 | 2.7163 | 7 | hepatocyte growth factor (hepapoletin A; scatter factor) | cytokine and growth factor | extracellular space | |
| HIG2 | 29923 | 218507_at | 8135915 | 2.6299 | 2.0696 | 7 | hypoxia-inducible protein 2 | | unknown | |
| HLA-DOB | 3112 | 206871_s_at | 8178833 | 2.9282 | 1.8281 | 6 | major histocompatibility complex, class U, DO beta | transmembranous receptor | cell membrane | |
| HLX | 3142 | 214438_at | 7909890 | 4.7545 | 1.972 | 1 | H2.0-like homeobox | transcription factor | nucleus | |
| HN1 | 51155 | 217755_at | 8018305 | 3.5232 | 2.6057 | 17 | hematological and neurological expressed | | nucleus | |
| HOMER3 | 9454 | 204647_at | 8035566 | 13.8417 | 4.0343 | 19 | homer homolog 3 (Drosophila) | signaling molecule | cell membrane | |
| HPGD | 3248 | 203914_x_at | 8103769 | 1.9977 | 5.1611 | 4 | hydroxyproataglandin dehydrogenase 15-(NAD) | | cytoplasm | cell cycle |
| HVCN1 | 84329 | 226879_at | 7966356 | 3.112 | 2.231 | 12 | hydrogen voltage-gated channel 1 | | unknown | |
| IDH1 | 3417 | 201193_at | 8058552 | 2.5681 | 2.1009 | 2 | isocitrate dehydrogenase 1 (NADP+), soluble | | cytoplasm | |
| IDH3A | 3419 | 202089_s_at | 7985134 | 3.9339 | 2.6845 | 15 | isocitrate dehydrogenase 3 (NAD+) alpha | | cytoplasm | |
| IER3 | 8870 | 201631_s_at | 8179704 | 2.9818 | 2.6543 | 6 | immediate early response 3 | | cytoplasm | apoptosis |
| IF130 | 10437 | 201422_at | 8026971 | 11.7514 | 3.0596 | 19 | Interferon, gamma-inducible protein 30 | | extracellular space | |
| IKIP | 121457 | 227295_at | 7965681 | 3.5724 | 1.8803 | 12 | IKK interacting protein | | unknown | |
| IL13RA1 | 3597 | 201887_at | 8169580 | 7.1957 | 2.4697 | X | interleukin 13 receptor, alpha 1 | transmembranous receptor | cell membrane | |
| IL2RG | 3561 | 204116_at | 8173444 | 2.2169 | 2.6537 | X | interleukin 2 receptor, gamma (severe combined Immunodeficiency) | transmembranous receptor | cell membrane | Immunity |
| IL3RA | 3563 | 206148_at | 8176323 | 3.392 | 2.9718 | X\|Y | interleukin 3 receptor, alpha (low affinity) | transmembranous receptor | cell membrane | |
| INHBA | 3624 | 210511_s_at | 8139207 | 7.886 | 1.977 | 7 | inhibin, beta A | cytokine and growth factor | extracellular space | |
| ITGB2 | 3689 | 1555349_a_at | 8070826 | 3.4371 | 2.3718 | 21 | integrin, beta 2 (complement component 3 receptor 3 and 4 | signaling molecule | cell membrane | cell adhesion, apoptosis |
| KIF2C | 11004 | 209408_at | 7901010 | 2.4566 | 2.3144 | 1 | kinesin family member 2C | | nucleus | |
| KYNU | 8942 | 217388_s_at | 8045539 | 21.2871 | 4.5148 | 2 | kynureninase(L-kynurenine hydrolase) | | cytoplasm | |
| LCMT2 | 9836 | 204012_s_at | 7988077 | 2.8266 | 1.8921 | 15 | leucine carboxyl methyltransferase 2 | | unknown | |
| LGALS1 | 3956 | 201105_at | 8072876 | 17.9891 | 7.0421 | 22 | lectin, galactoside-binding, soluble, 1 | | extracellular space | apoptosis |
| LPXN | 9404 | 216250_s_at | 7948332 | 6.1566 | 5.0537 | 11 | leupaxin | signaling molecule | cytoplasm | cell adhesion |
| LRG1 | 116844 | 228648_at | 8032834 | 5.7066 | 2.2013 | 19 | leucine-rich alpha-2-glycoprotein 1 | | extracellular space | |
| LY86 | 9450 | 205859_at | 8116734 | 9.8638 | 11.3294 | 6 | lymphocyte antigen 86 | | cell membrane | immunity, apoptosis |

**[Table 1-5]**

| | | | | | | | | | | |
|---|---|---|---|---|---|---|---|---|---|---|
| MAMDC2 | 256691 | 228885_at | 8155754 | 50.0231 | 1.8485 | 9 | MAM domain containing 2 | | extracellular space | |
| ME1 | 4199 | 204059_s_at | 8127854 | 3.167 | 6.0952 | 6 | mallo enzyme 1, NADP(+)-dependent, cytosolic | | cytoplasm | |
| MGAT4A | 11320 | 226039_at | 8054135 | 2.488 | 2.145 | 2 | mannosyl (alpha-1,3-)-glycoprotein beta-1,4-N- | | extracellular space | |
| MIRN21 | 406991 | 224917_at | 8008885 | 7.1437 | 2.2647 | 17 | microRNA 21 | | unknown | |
| MKKS | 8195 | 218138_at | 8064967 | 5.0082 | 2.1826 | 20 | McKusick-Kaufman syndrome | | cytoplasm | |
| MNDA | 4332 | 204959_at | 7906377 | 7.9908 | 6.7427 | 1 | myeloid cell nuclear differentiation antigen | | nucleus | |
| MPO | 4353 | 203949 at | 8016932 | 3.4405 | 2.9167 | 17 | myeloperoxidase | | cytoplasm | apoptosis |
| MS4A3 | 932 | 210254_at | 7940216 | 2.9166 | 6.6488 | 11 | membrane-spanning 4-domains, subfamily A, member 3 | signaling molecule | cytoplasm | |
| MTHFD2 | 10797 | 201761_at | 8042830 | 2.7123 | 1.9426 | 2 | methylenetetrahydrofolate dehydrogenase (NADP+ dependent) 2, | | cytoplasm | |
| MYC | 4609 | 202431_s_at | 8148317 | 4.8528 | 1.9292 | 8 | v-myo myelocytomatosis viral oncogene homolog (avian) | transcription factor | nucleus | |
| MYO1B | 4430 | 212364_at | 8047127 | 3.1023 | 1.9101 | 2 | myosin IB | | cytoplasm | |
| MYO1F | 4542 | 213733_at | 8033605 | 3.93 | 2.5391 | 19 | myosin IF | | cytoplasm | |
| NAGA | 4668 | 202943_s_at | 8076403 | 2.7565 | 2.4116 | 22 | N-acetylgalactosaminidase, alpha- | | cytoplasm | |
| NAIP | 4671 | 239944_at | 8177527 | 3.9106 | 2.0172 | 5 | NLR family, apoptosis inhibitory protein | | cytoplasm | apoptosis |
| NCF2 | 4688 | 209949_at | 7922773 | 8.0756 | 3.4526 | 1 | neutrophil cytosollo factor 2 | | cytoplasm | |
| NCF4 | 4689 | 205147_x_at | 8072744 | 3.0753 | 3.3081 | 22 | neutrophil cytosolic factor 4, 40kDa | | cytoplasm | immunity |
| NDUFAF1 | 51103 | 204125_at | 7987642 | 4.6031 | 2.1624 | 15 | NADH dehydrogenase (ubiquinone) 1 alpha subcomplex, assembly factor | | cytoplasm | |
| NEK6 | 10783 | 223159_s_at | 8157761 | 5.1566 | 3.4558 | 9 | NIMA (never in mitosis gene a)-related kinase 6 | signaling moleoule | nucleus | cell cycle, apoptosis |
| NP | 4860 | 201695_a_at | 7973067 | 8.9631 | 2.1755 | 14 | nucleoside phosphorylase | | nucleus | |
| NRIP3 | 56675 | 219657_s_at | 7940446 | 3,2732 | 3.6133 | 11 | nuclear receptor Interacting protein 3 | | unknown | |
| OBFG2A | 64859 | 222872_x_at | 8047161 | 13.5591 | 2.7032 | 2 | oligonucleotide/oligosaccharide-binding fold containing 2A | | nucleus | |
| P2RY14 | 9934 | 206637_at | 8091511 | 3.0798 | 2.4295 | 3 | purinergio receptor P2Y, G-protein coupled, 14 | transmembranous receptor | cell membrane | |
| P2RY5 | 10161 | 218588_at | 7971565 | 1.5532 | 2.7233 | 13 | purinergio receptor P2Y, G-protein coupled, 5 | transmembranous receptor | cell membrane | |
| PAK1IP1 | 55003 | 218886_at | 8116848 | 4.0989 | 2.4834 | 6 | PAK1 interacting protein 1 | signaling molecule | nucleus | |
| PARP8 | 79668 | 219033_at | 8105191 | 7.2902 | 2.9745 | 5 | poly (ADP-ribose) polymerase family, member 8 | | nucleus | |
| PDE9A | 5152 | 205593_s_at | 8068833 | 5.8044 | 3.5595 | 21 | phosphodiesterase 9A | signaling molecule | cytoplasm | |
| PDK1 | 5163 | 226452_at | 8046408 | 2.6927 | 2.4033 | 2 | pyruvate dehydrogenase kinase, isozyme 1 | signaling molecule | cytoplasm | |
| PDLIM1 | 9124 | 208690_s_at | 7935180 | 15.0721 | 1.5551 | 10 | PDZ and LIM domain 1 | | cytoplasm | |
| PDSS1 | 23590 | 220865_s_at | 7926807 | 2.7366 | 2.4594 | 10 | prenyl (decaprenyl) diphosphate synthase, subunit 1 | | unknown | |
| PGM2 | 55276 | 225366_at | 8094556 | 3.8421 | 2.1135 | 4 | phosphoglucomutase 2 | | cytoplasm | |
| PIGK | 10026 | 209707_at | 7917088 | 4.7506 | 2.9006 | 1 | phosphatidylinositol glycan anchor biosynthesis, class K | | cytoplasm | |
| PIWIL4 | 143689 | 230480_at | 7943240 | 3.308 | 1.9009 | 11 | piwi-like 4 (Drosophila) | | unknown | |
| PLAUR | 5329 | 210845_s_at | 8037374 | 6.5367 | 1.697 | 19 | plasminogen activator, urokinase receptor | transmembranous receptor | cell membrane | |

**[Table 1-6]**

| | | | | | | | | | | |
|---|---|---|---|---|---|---|---|---|---|---|
| PPBP | 5473 | 214146_s_at | 8100971 | 12.1498 | 1.5966 | 4 | pro-platelet basic protein (chemokine (C-X-G motif) ligand 7) | cytokine and growth factor | extracellular space | |
| PPCDC | 60490 | 219066_at | 7984943 | 3.3971 | 2.0648 | 15 | phosphopantothenoylcysteine decarboxylase | | unknown | |
| PPIF | 10105 | 201489_at | 7928589 | 5.105 | 2.4099 | 10 | peptidylprolyl Isomerase F (cyclophllin F) | | cytoplasm | |
| PRAME | 23532 | 204086_at | 8074856 | 2.9345 | 7.1984 | 22 | preferentially expressed antigen In melanoma | | nucleus | |
| PRG2 | 5553 | 211743_s_at | 7948221 | 5.7443 | 5.1313 | 11 | proteoglycan 2, bone marrow (natural killer cell activator, | | extracellular space | |
| PRKAR1A | 5573 | 200604_s_at | 8009457 | 2.5728 | 1.9221 | 17 | protein kinase, cAMP-dependent, regulatory, type I, alpha (tissue | signaling moleoule | cytoplasm | |
| PRKCD | 5580 | 202545_at | 8080487 | 11.8884 | 4.4878 | 3 | protein kinase C, delta | signaling molecule | cytoplasm | |
| PRSS21 | 10942 | 220051_at | 7992722 | 2.8945 | 2.4783 | 16 | protease, serine, 21 (testisin) | | extracellular space | |
| PTH2R | 5746 | 206772_at | 8047910 | 32.9485 | 2.6488 | 2 | parathyroid hormone 2 receptor | transmembranous receptor | cell membrane | |
| PTX3 | 5808 | 208157_at | 8083594 | 1.371 | 1.7203 | 3 | pentraxin-related gene, rapidly induced by IL-1 beta | | extracellular space | |
| PUS7 | 54517 | 218984_at | 8142061 | 3.473 | 2.0097 | 7 | pseudouridylate synthase 7 homolog (S. cerevisiae) | | unknown | |
| PXK | 54899 | 1552275_s_at | 8080781 | 2.9549 | 2.2995 | 3 | PX domain containing serine/threonine kinase | signaling molecule | cytoplasm | |
| PYHIN1 | 149628 | 240413_at | 7906386 | 3.6814 | 1.9866 | 1 | pyrin and HIN domain family, member 1 | | nucleus | cell cycle |
| RAB20 | 55647 | 219622_at | 7972805 | 4.2758 | 1.9982 | 13 | RAB20, member RAS oncogene family | signaling molecule | cytoplasm | |
| RAB8A | 4218 | 208819_at | 8026520 | 3.089 | 2.0472 | 19 | RAB8A, member RAS oncogene family | signaling molecule | cytoplasm | |
| RABIF | 5877 | 204478_s_at | 7923483 | 6.673 | 1.9163 | 1 | RAB interacting factor | signaling moleoule | unknown | |
| RASGRP3 | 25780 | 205801_s_at | 8041422 | 12.2403 | 2.3489 | 2 | RAS guanyl releasing protein 3 (calcium and DAG-regulated) | signaling molecule | cytoplasm | |
| RASSF4 | 83937 | 226436_at | 7927186 | 5.1219 | 1.8566 | 10 | Ras association (RaIGDS/AF-6) domain family member 4 | | unknown | cell cycle |
| REEP5 | 7905 | 208873_s_at | 8113542 | 1.9895 | 2.4938 | 5 | receptor accessory protein 5 | | extracellular space | |
| RGS18 | 64407 | 223809_at | 7908376 | 18.2071 | 3.0532 | 1 | regulator of G-protein signaling 18 | signaling molecule | cytoplasm | |
| RNASE2 | 6036 | 206111_at | 7973110 | 6.2056 | 30.3509 | 14 | ribonuclease, RNase A family, 2 (liver, eosinophil-derived | | extracellular space | |
| RPP40 | 10799 | 213427_at | 8123717 | 2.5261 | 2.3867 | 6 | ribonuclease P/MRP 40kDa subunit | | nucleus | |
| RRM2 | 6241 | 201890_at | 8040223 | 1.8022 | 1.9629 | 2 | ribonucleotide reductase M2 polypeptide | | cytoplasm | |
| RXFP1 | 59350 | 231804_at | 8098060 | 8.4366 | 4.7218 | 4 | relaxin/insulin-like family peptide receptor 1 | transmembranous receptor | cell membrane | |
| S100A11 | 6282 | 200660_at | 7920128 | 2.6989 | 1.9757 | 1 | S100 calcium binding protein A11 | signaling molecule | cytoplasm | |
| S100A16 | 140576 | 227998_at | 7920291 | 6.5974 | 5.2295 | 1 | S100 calcium binding protein A16 | | nucleus | |
| S100AB | 6279 | 202917_s_at | 7920244 | 3.7254 | 4.1401 | 1 | S100 calcium binding protein A8 | | cytoplasm | |
| S100P | 6286 | 204351_at | 8093950 | 2.8439 | 4.35 | 4 | S100 calcium binding protein P | | cytoplasm | |
| S100Z | 170591 | 1554876_a_at | 8106411 | 2.5656 | 3.2588 | 5 | S100 calcium binding protein Z | | unknown | |
| SAMHD1 | 25939 | 204502_at | 8066117 | 3.5478 | 3.315 | 20 | SAM domain and HD domain 1 | | nucleus | immunity |
| SH2D1A | 4068 | 210116_at | 8169792 | 5.4768 | 4.944 | X | SH2 domain protein 1A | | cytoplasm | |
| SLC31A2 | 1318 | 204204_at | 8157264 | 6.5551 | 1.9871 | 9 | solute carrier family 31 (copper transporters), member 2 | | cell membrane | |
| SLC43A3 | 29015 | 213113_s_at | 7948229 | 4.0265 | 2.5036 | 11 | solute carrier family 43, member 3 | | extracellular space | |

**[Table 1-7]**

| | | | | | | | | | | |
|---|---|---|---|---|---|---|---|---|---|---|
| SLG6A6 | 6533 | 211030_s_at | 8078014 | 3.3096 | 1.9332 | 3 | solute carrier family 6 (neurotransmitter transporter, | | cell membrane | |
| SLC7A6 | 9057 | 203579_s_at | 7996772 | 2.537 | 2.0624 | 16 | solute carrier family 7 (cationic amino acid transporter, y+ | | cell membrane | |
| SPECS2 | 9789 | 201239_s_at | 7914180 | 2.8247 | 2.8859 | 11 | signal peptidase complex subunit 2 homolog (S. cerevisiae) | | cytoplasm | |
| SPPL2A | 84888 | 226353_at | 7988753 | 3.6826 | 3.1645 | 15 | signal peptide peptidase-like 2A | | unknown | |
| STX7 | 8417 | 212631_at | 8129590 | 3.044 | 1.957 | 6 | syntaxin 7 | | cell membrane | |
| SUCNR1 | 56670 | 223939_at | 8083422 | 10.3593 | 8.9094 | 3 | succinate receptor 1 | transmembranous receptor | cell membrane | |
| TACSTD2 | 4070 | 202286_s_at | 7916584 | 4.5271 | 2.3856 | 1 | tumor-associated calcium signal transducer 2 | | cell membrane | |
| TESC | 54997 | 218872_at | 7966749 | 6.7818 | 4.194 | 12 | tescalcin | | unknown | |
| THEX1 | 90459 | 226416_at | 8144516 | 3.539 | 2.084 | 8 | three prime histone mRNA exonuclease 1 | | unknown | |
| TIMP1 | 7076 | 201666_at | 8167185 | 2.6582 | 1.8176 | X | TIMP metallopeptidase inhibitor 1 | | extracellular space | |
| M4SF1 | 4071 | 215034_s_at | 8091411 | 13.9334 | 3.1159 | 3 | transmembrane 4 L six family member 1 | | cell membrane | |
| M9SF1 | 10548 | 209149_s_at | 7978166 | 3.8307 | 1.9283 | 14 | transmembrane 9 superfamily member 1 | | cell membrane | |
| TMEM30A | 55754 | 232591_s_at | 8127637 | 3.436 | 1.8579 | 6 | transmembrane protein 30A | | unknown | |
| TMEM33 | 55161 | 218465_at | 8094830 | 2.7565 | 2.4022 | 4 | transmembrane protein 33 | | unknown | |
| TNF | 7124 | 207113_s_at | 8179263 | 5.16 | 3.3831 | 6 | tumor necrosis factor (TNF superfamily, member 2) | cytokine and growth factor | extracellular space | immunity, apoptosis |
| TNFRSF4 | 7293 | 214228_x_at | 1911413 | 4.2204 | 2.4055 | 1 | tumor necrosis factor receptor superfamily, member 4 | transmembranous receptor | cell membrane | immunity |
| TNFSF13B | 10673 | 223501_at | 7969986 | 9.7537 | 2.3209 | 13 | tumor necrosis factor (ligand) superfamily, member 13b | cytokine and growth factor | extracellular space | immunity |
| TRIP 13 | 9319 | 204033_at | 8104234 | 2.146 | 2.0662 | 5 | thyroid hormone receptor Interactor 13 | | cytoplasm | |
| TUBB6 | 84617 | 209191_at | 8020220 | 5.4543 | 1.8033 | 18 | tubulin, beta 6 | | cytoplasm | |
| XNDC1 | 81542 | 208097 s at | 7974303 | 2.8632 | 1.9163 | 14 | thioredoxin domain containing 1 | | cytoplasm | apoptosis |
| TXNL4B | 54957 | 222748_s_at | 8002660 | 4.1397 | 1.9453 | 16 | thioredoxin-like 4B | | nucleus | cell cycle |
| TYROBP | 7305 | 204122_at | 8036224 | 21.8206 | 5.4076 | 19 | TYRO protein tyrosine kinase binding protein | signaling molecule | cell membrane | |
| UBASH3B | 84959 | 238587_at | 7944722 | 3.5884 | 2.858 | 11 | ubiquitin associated and SH3 domain containing, B | | unknown | |
| UGCG | 7357 | 221765_at | 8157216 | 4.0106 | 2.3729 | 9 | glucosyltransferase | | cytoplasm | |
| UTS2 | 10911 | 220784_s_at | 7912136 | 5.3996 | 6.4651 | 1 | urotensin 2 | | extracellular space | |
| VNN1 | 8876 | 205844_at | 8129618 | 11.9292 | 5.0877 | 6 | vanin 1 | | cell membrane | immunity, apoptosis, cell adhesion |
| VSTM1 | 284415 | 236818_at | 8039109 | 2.8594 | 5.2732 | 19 | V-set and transmembrane domain containing 1 | | unknown | |
| WDR4 | 10785 | 241937_s_at | 8070615 | 2.8509 | 2.2356 | 21 | WD repeat domain 4 | | nucleus | |
| WIT1 | 51352 | 206954_at | 7939131 | 2.8415 | 2.6555 | 11 | Wilms tumor upstream neighbor 1 | | unknown | |
| WSB2 | 55884 | 201760_s_at | 7966829 | 3.5848 | 1.9757 | 12 | WD repeat and SOCS box-containing 2 | | unknown | |
| WT1 | 7490 | 206067_s_at | 7947363 | 93.6087 | 1.7707 | 11 | Wilms tumor 1 | transcription factor | nucleus | cell cycle |
| ZNF253 | 56242 | 242919_at | 8027241 | 2.6622 | 2.176 | 19 | zinc finger protein 253 | | nucleus | |
| ZWINT | 11130 | 204026_s_at | 7933707 | 1.9796 | 2.3419 | 10 | ZW10 interactor | | nucleus | cell cycle |

When the test method of the present invention is intended to more clearly distinguish between LSCs and HSCs, it is preferable that the following marker genes (2), for example, out of the above-described marker genes (1), be used as an index. In this mode of embodiment, the marker genes (2) consist of 2 to 58 genes, more preferably consist of 3 or more, 5 or more, 10 or more, 15 or more, 20 or more, or 25 or more, genes. When the test method of the present invention is intended to still more clearly distinguish between LSCs and HSCs, it is preferable that the following marker genes (3), out of the marker genes (2), be used as an index. The marker genes (3) are more preferable because normally 5 times or higher differential expression is observed in LSCs than in HSCs. In this mode of embodiment, the marker genes (3) consist of 2 to 35 genes, more preferably consist of 3 or more, 5 or more, 10 or more, 15 or more, 20 or more, or 25 or more, genes.

### Marker genes (2):

cell membrane- or extracellularly-localized genes consisting of ADFP, ALOX5AP, CACNB4, CCL5, CD33, CD3D, CD93, CD97, CLEC12A, DOK2, FCER1G, FCGR2A, FUCA2, GPR34, GPR84, HCST, HGF, HOMER3, IL2RA, IL2RG, IL3RA, ITGB2, LGALS1, LRG1, LY86, MGAT4A, P2RY5, PRSS21, PTH2R, RNASE2, SLC43A3, SUCNR1, TIMP1, TNF, TNFRSF4, TNFSF13B, TYROBP and VNN1; cell cycle-related genes consisting of ZWINT, NEK6 and TXNL4B; an apoptosis-related gene consisting of BIK; signaling-related genes consisting of AK5, ARHGAP18, FYB, HCK, LPXN, PDE9A, PDK1, PRKCD, RAB20, RAB8A and RABIF; transcription factor genes consisting of WT1 and HLX; and other genes consisting of CYBB, CTSC and NCF4.

### Marker genes (3):

cell membrane- or extracellularly-localized genes consisting of ALOX5AP, CACNB4, CCL5, CD33, CD3D, CD93, CD97, CLEC12A, DOK2, FCGR2A, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4.

Although the subject in the test method of the present invention is not particularly limited, as far as it is a mammal, including a human, a human suspected of suffering the initial onset or a recurrence of leukemia is preferred.

The biological sample to be measured by the test method of the present invention is not particularly limited, as far as it can be collected from a mammal, preferably from a human; examples include humoral samples such as blood, bone marrow fluid, and lymph fluid, and solid samples such as lymph nodes, blood vessels, bone marrow, brain, spleen, and skin.

In the test method of the present invention, the expression level of a marker gene is measured for a transcription product or translation product of the gene as an analyte. When the transcription product is the analyte, RNA can be isolated from the biological sample by a conventional method. Ordinary methods for RNA extraction are well known in the relevant technical field, and are disclosed in standard textbooks of molecular biology, including Ausubel et al., Current Protocols of Molecular Biology, John Wiley and Sons (1997) and the like. Specifically, isolation of RNA can be achieved using purification kits, buffer solution sets, and proteases obtained from their manufacturers, such as Qiagen, as directed by the manufacturers.

The method of measuring the expression level of a marker gene for a transcription product as an analyte is not particularly limited; available methods include Northern blotting and in situ hybridization (Parker & Barnes, Methods in Molecular Biology 106: 247-283 (1999)); RNase protection assay (Hod, Biotechniques 13: 852-854 (1992)); reverse transcription polymerase chain reaction (RT-PCR) (Weis et al., Trends in Genetics 8: 263-264 (1992)); realtime quantitative RT-PCR (Held et al., Genome Research 6: 986-994 (1996)); microarray analysis and the like. Microarray analysis can be performed using the Affymetrix GeneChip technique, the microarray technique of Agilent Technologies or the microarray technique of Incyte with a commercially available apparatus, as directed by the manufacturer. Details of realtime quantitative RT-PCR are described in Examples below. Examples of the base sequences of primers and probes that are suitably used for realtime quantitative RT-PCR are listed in Table 3 and the sequence listing.

When the translation product of a marker gene is the analyte, protein can be isolated from the biological sample according to a conventional method. Ordinary methods for protein extraction are well known in the relevant technical field, and are disclosed in standard textbooks of molecular biology, including Ausubel et al., Current Protocols of Molecular Biology, John Wiley and Sons (1997) and the like. Isolation of protein can be achieved using purification kits, buffer solution sets, and protease inhibitors obtained from their manufacturers, as directed by the manufacturers.

The method of measuring the expression level of a marker gene for a translation product as an analyte is not particularly limited; available methods include the immunohistochemical method, the proteomics method and the like. The immunohistochemical method comprises detecting the expression using an antibody specific for each marker gene product. Protocols and kits for the immunohistochemical method are well known in the relevant technical field, and are commercially available. The proteomics method comprises examining overall changes in protein expression in a certain sample. The proteomics method generally comprises the following steps: (1) separation of various proteins in the sample by 2-D gel electrophoresis (2-D PAGE), (2) identification of the various proteins recovered from this gel by, for example, mass analysis or N-terminal sequencing, and (3) data analysis using bioinfbrmatics. The proteomics method is a useful method for supplementing other gene expression profiling methods, and can be used alone, or in combination with another method, to detect products of marker genes of the present invention. When a cell surface marker is the target, a measuring method using flow cytometry is possible.

### (2) Step of comparing the expression levels obtained in the measuring step with a reference value

When the results of measurements of the expression levels of 2 to 218 kinds of marker genes in a biological sample show that the expression levels of 2 kinds or more thereof are significantly higher than reference values (gene expression differs about 2 fold or more, preferably about 4 fold or more, more preferably about 6 fold or more, most preferably about 10 fold or more), the possible presence of a leukemic stem cell in the sample or the subject's body is suggested. Here, useful reference values include comparator values such as mean expression levels for healthy persons and mean levels for the subject before onset. The suggestion of the possible presence of leukemic stem cell leads to prediction of the initial onset or a recurrence of leukemia in the subject. It is preferable that the presence or absence of the initial onset or a recurrence of leukemia be checked by another test.

In the test method of the present invention, when the results of measurements of the expression levels of 2 to 218 kinds of marker genes in a biological sample show that the expression levels of 2 kinds or more thereof are significantly higher than reference values (gene expression differs about 2 fold or more, preferably about 4 fold or more, more preferably about 6 fold or more, most preferably about 10 fold or more), the possible presence of a leukemic stem cell in the sample or the body of the source from which the sample has been collected is suggested. Here, useful reference values include comparator values such as mean expression levels for healthy persons and mean expression level for the subject before onset. In this case, the suggestion of the possible presence of a leukemic stem cell leads to prediction that the treatment is not completely effective on the cancer in the leukemia patient. Conversely, when the expression levels of the aforementioned 2 kinds or more are significantly lower (for example, substantially zero), it can be predicted that leukemic stem cells are absent in the sample. In this case, it is thought that the treatment of leukemia eliminated leukemic stem cells and is effective. Furthermore, it is preferable that the test method be combined with other examinations to achieve multi-angle confirmation of a therapeutic effect on leukemia.

As stated above, by applying the test method of the present invention, it is possible to detect leukemic stem cells in a living organism before leukemia occurs initially or recurs, and predict the onset. Alternatively, it is also possible to detect the onset of leukemia in the initial stage and lead to early treatment of cancer patients. Furthermore, it is also possible to evaluate the therapeutic effect on leukemia patients with the presence or absence of leukemic stem cells as an index.

### (Therapeutic agent)

Also disclosed is a therapeutic agent for acute myeloid leukemia that targets leukemic stem cells, comprising as an active ingredient a substance capable of suppressing the expression of a leukemic stem cell marker gene or a substance capable of suppressing the activity of a translation product of the gene.

Molecular targets for the therapeutic agent are the above-described leukemic stem cell marker genes, and any marker may be selected according to the purpose of treatment. When the therapeutic agent targets stem cells, out of leukemic stem cells, that are present in bone marrow niches, are in the stationary phase of cell cycle, and are resistant to anticancer agents, it is recommended that a substance capable of suppressing the expression of genes selected from the group consisting of AK5, BIK, DOK2, FCGR2A, IL2RA, LRG1, SUCNR1 and WT1 (hereinafter also referred to as marker genes (4)) or a substance capable of suppressing the activity of a translation product of the gene be selected. By selecting 2 to 8 (preferably 2 to 5) out of the eight genes constituting the marker genes (4) and using them as molecular targets, it is highly likely possible to exterminate leukemic stem cells of a large number of patients. Therefore, at least one active ingredient is contained in the therapeutic agent, and it is preferable that two or more be combined according to the purpose of treatment. Two or more active ingredients may be contained in a single pharmaceutical preparation, or may be contained in separate pharmaceutical preparations.

Described below are active ingredients.

Substances capable of suppressing the expression of a leukemic stem cell marker gene include, for example, antisense nucleic acids, RNAi-inducible nucleic acids and the like.

Substances capable of suppressing the activity of a translation product of a leukemic stem cell marker gene include, for example, aptamers, antibodies and the like. The substance may be an inhibitory substance that acts directly or indirectly on each marker.

Described below are active ingredients of the therapeutic agent.

### 1. Antisense nucleic acid

The kind of the antisense nucleic acid may be DNA or RNA, or a DNA/RNA chimera. The antisense nucleic acid may be one having a natural type phosphoric acid diester bond, or a modified nucleotide such as of the thiophosphate type (P=O in phosphate linkage replaced with P=S), 2'-O-methyl type and the like, which are stable to decomposing enzymes. Other important factors for the designing of antisense nucleic acids include increases in water-solubility and cell membrane permeability and the like; these can also be cleared by choosing appropriate dosage forms such as those using liposome or microspheres. The length of the antisense nucleic acid is not particularly limited, as far as the antisense nucleic acid is capable of specifically hybridizing with the transcription product; the antisense nucleic acid may be a sequence comprising about 15 nucleotides for the shortest, or comprising a sequence complementary to the entire sequence of the transcription product for the longest. Taking into account the issues of the ease of synthesis, antigenicity and the like, oligonucleotides consisting of, for example, about 15 or more nucleotides, preferably about 15 to about 100 nucleotides, more preferably about 18 to about 50 nucleotides, can be mentioned as examples. Furthermore, the antisense nucleic acid may be one that not only hybridizes with the transcription product to inhibit the translation, but also is capable of binding to a double-stranded DNA to form a triple strand (triplex) to inhibit the transcription into mRNA.

### 2. RNAi-inducible nucleic acid

An RNAi-inducible nucleic acid refers to a polynucleotide, preferably an RNA, capable of inducing the RNA interference (RNAi) effect when introduced into cells. The RNAi effect refers to the phenomenon in which a double-stranded RNA comprising the same nucleic acid sequence as that of mRNA, or a partial sequence thereof, suppresses the expression of the mRNA. To obtain the RNAi effect, it is preferable to use, for example, a double-stranded RNA having the same nucleic acid sequence as that of the target mRNA comprising at least 19 continuous bases (or a partial sequence thereof). The double-stranded structure may be configured by different strands, or may be a double strand conferred by a stem-loop structure of one RNA. Examples of RNAi-inducing nucleic acids include siRNAs, miRNAs and the like, with preference given to siRNAs. The siRNA is not particularly limited, as far as it can induce an RNAi, and the siRNA can be, for example, 19 to 27 bases long, preferably 21 to 25 bases long.

### 3. Aptamer

An aptamer refers to a polynucleotide having a binding activity (or inhibitory activity) on a specified target molecule. An aptamer is an RNA, a DNA, a modified nucleotide or a mixture thereof. The aptamer can be in a linear or circular form. The length of the aptamer is not particularly limited, and is normally about 16 to about 200 nucleotides; for example, the length is about 100 nucleotides or less, preferably about 50 nucleotides or less, and more preferably about 40 nucleotides or less. The length of the aptamer may be, for example, about 18, about 20, about 25 or about 30, nucleotides or more. The aptamer, for increasing the bindability, stability, drug delivering quality and the like, may be one wherein a sugar residue (e.g., ribose) of each nucleotide is modified. Examples of portions of the sugar residue where it is modified include ones wherein the oxygen atom at the 2'-position, 3'-position and/or 4'-position of the sugar residue is replaced with another atom and the like. Examples of types of modifications include fluorination, O-alkylation, O-allylation, S-alkylation, S-allylation and amination (see, e.g., Sproat et al., (1991) Nucle. Acid. Res. 19, 733-738; Cotton et al., (1991) Nucl. Acid. Res. 19, 2629-2635). The aptamer may also be one wherein a purine or pyrimidine is altered. Examples of such alterations include alteration of the 5-position pyrimidine, alteration of the 8-position purine, alteration with an exocyclic amine, substitution with 4-thiouridine, and substitution with 5-bromo or 5-iodo-uracil. The phosphate group contained in the aptamer of the present invention may be altered to make it resistant to nucleases and hydrolysis. For example, the phosphate group may be substituted with a thioate, a dithioate or an amidate. An aptamer can be prepared according to available reports (for example, Ellington et al., (1990) Nature, 346, 818-822; Tuerk et al., (1990) Science, 249, 505-510).

### 4. Antibody

The antibody may be a polyclonal antibody (antiserum) or a monoclonal antibody, and can be prepared by a commonly known immunological technique. Although the monoclonal antibody may be of any isotype, IgG, IgM, IgA, IgD, IgE, or the like, IgG or IgM is preferable.

For example, the polyclonal antibody can be acquired by subcutaneously or intraperitoneally administering the above-described antigen (as required, may be prepared as a complex crosslinked to a carrier protein such as bovine serum albumin or KLH (Keyhole Limpet Hemocyanin)), along with a commercially available adjuvant (for example, Freund's complete or incomplete adjuvant), to an animal about 2 to 4 times at intervals of 2 to 3 weeks (the antibody titer of partially drawn serum has been determined by a known antigen-antibody reaction and its elevation has been confirmed in advance), collecting whole blood about 3 to 10 days after final immunization, and purifying the antiserum. Animals to receive the antigen include mammals such as rats, mice, rabbits, goat, guinea pigs, and hamsters.

The monoclonal antibody can also be prepared by cell fusion. For example, the above-described antigen, along with a commercially available adjuvant, is subcutaneously or intraperitoneally administered to a mouse 2 to 4 times, and 3 days after final administration, the spleen or lymph nodes are collected, and leukocytes are collected. These leukocytes and myeloma cells (for example, NS-1, P3X63Ag8 and the like) are cell-fused to obtain a hybridoma that produces a monoclonal antibody against the factor. This cell fusion may be performed by the PEG method or the voltage pulse method. A hybridoma that produces the desired monoclonal antibody can be selected by detecting an antibody that binds specifically to the antigen in the culture supernatant, using a widely known EIA or RIA method and the like. Cultivation of the hybridoma that produces the monoclonal antibody can be performed in vitro, or in vivo such as in ascitic fluid of a mouse or rat, preferably a mouse, and the antibody can be acquired from the culture supernatant of the hybridoma or the ascitic fluid of the animal.

The antibody may be a chimeric antibody, a humanized antibody or a human antibody.

A chimeric antibody means a monoclonal antibody derived from immunoglobulins of animal species having mutually different variable regions and constant regions. For example, the chimeric antibody can be a mouse/human chimeric monoclonal antibody whose variable region is a variable region derived from a mouse immunoglobulin, and whose constant region is a constant region derived from a human immunoglobulin. The constant region derived from a human immunoglobulin has an amino acid sequence unique depending on the isotype, IgG, IgM, IgA, IgD, IgE or the like, and the constant region of a recombinant chimeric monoclonal antibody in the present invention may be the constant region of a human immunoglobulin belonging to any isotype. The constant region of human IgG is preferable.

A chimeric antibody can be prepared by a method known per se. For example, a mouse/human chimeric monoclonal antibody can be prepared according to available reports (e.g., Jikken Igaku (extra issue), Vol. 6, No.10, 1988 and JP-B-HEI-3-73280). In detail, a mouse/human chimeric monoclonal antibody can be prepared by inserting the C_{H} gene acquired from the DNA that encodes a human immunoglobulin (C gene that encodes H chain constant region) downstream of the active V_{H} gene acquired from the DNA that encodes a mouse monoclonal antibody, isolated from a hybridoma that produces the mouse monoclonal antibody (rearranged VDJ gene that encodes H chain variable region), and inserting the C_{L} gene acquired from the DNA that encodes a human immunoglobulin (C gene that encodes L chain constant region) downstream of the active V_{L} gene acquired from the DNA that encodes a mouse monoclonal antibody, isolated from the hybridoma (rearranged VJ gene that encodes L chain variable region), into one or separate expression vectors in a way that allows the expression of each gene, transforming a host cell with the expression vector, and culturing the transformant cell.

A humanized antibody means a monoclonal antibody prepared by a gene engineering technique, for example, a human type monoclonal antibody wherein some or all of the complementarity-determining regions of the ultra-variable region thereof are derived from a mouse monoclonal antibody, and the framework region of the variable region thereof and the constant region thereof are derived from a human immunoglobulin. The complementarity-determining regions of the ultra-variable region are three regions that are present in the ultra-variable region in the variable region of the antibody, and that complementarily bind directly to the antigen (complementarity-determining regions; CDR1, CDR2, CDR3), and the framework regions of the variable region are four relatively highly conserved regions interposing the front and back of the three complementarity-determining regions (frameworks; FR1, FR2, FR3, FR4). Hence, a humanized antibody means, for example, a monoclonal antibody wherein all regions other than some or all of the complementarity-determining regions of the ultra-variable region of a mouse monoclonal antibody are replaced with corresponding regions of a human immunoglobulin.

A humanized antibody can be prepared by a method known per se. For example, a recombinant humanized antibody derived from a mouse monoclonal antibody can be prepared according to available reports (e.g., Japanese Patent Application Kohyo Publication No. HEI-4-506458 and JP-A-SHO-62-296890). In detail, from a hybridoma that produces a mouse monoclonal antibody, at least one mouse H chain CDR gene and at least one mouse L chain CDR gene corresponding to the mouse H chain CDR gene are isolated, and from a human immunoglobulin gene, the human H chain gene that encodes all regions other than the human H chain CDR corresponding to the mouse H chain CDR and the human L chain gene that encodes all regions other than the human L chain CDR corresponding to the mouse L chain CDR are isolated. The mouse H chain CDR gene and human H chain gene isolated are introduced into an appropriate expression vector expressibly; likewise, the mouse L chain CDR gene and the human L chain gene are introduced into another appropriate expression vector expressively. Alternatively, the mouse H chain CDR gene/human H chain gene and the mouse L chain CDR gene/human L chain gene can be introduced into the same expression vector expressively. By transforming a host cell with the expression vector thus prepared, it is possible to obtain a cell that produces a humanized antibody, and by culturing the cell, the desired humanized antibody can be obtained from the culture supernatant.

A human antibody means an antibody wherein all regions comprising the variable regions and constant regions of the H chain and L chain constituting an immunoglobulin are derived from the gene that encodes a human immunoglobulin.

A human antibody can be prepared by a method known per se. For example, a human antibody can be produced by immunologically sensitizing with an antigen a transgenic animal prepared by incorporating at least a human immunoglobulin gene into a gene locus of a non-human mammal such as a mouse, in the same way as the above-described method of preparing a polyclonal antibody or a monoclonal antibody. For example, a transgenic mouse that produces a human antibody can be prepared according to available reports (Nature Genetics, Vol.15, p.146-156, 1997; Nature Genetics, Vol.7, p.13-21, 1994; Japanese Patent Application Kohyo Publication No. HEI-4-504365; International Patent Application Publication WO94/25585; Nature, Vol.368, p.856-859, 1994; and Japanese Patent Application Kohyo Publication No. HEI-6-500233).

The antibody may be a part of the above-mentioned antibody (e.g., monoclonal antibody). The antibody may be a fragment such as F(ab')₂, Fab', Fab, Fv and the like, a conjugate molecule prepared by genetic engineering such as scFv, scFv-Fc, minibody, diabody and the like, or a derivative thereof, which is modified by a molecule and the like having a protein stabilizing action such as polyethylene glycol (PEG) and the like, and the like.

The above-described antibody may be in the form of an immunoconjugate bound with various anticancer substances and the like by a conventional method. In this case, the antibody functions as a drug delivery system for delivering an anticancer agent to LSCs. Anticancer substances to be combined include, but are not limited to, cisplatin, carboplatin, cyclophosphamide, melphalan, carmusulin, methotrexate, 5-fluorouracil, cytarabine (AraC), mercaptopurine, daunorubicin, idarubicin, mitoxantrone, thioguanine, azacitidine, amsacrine, doxorubicin, tretinoin, allopurinol, prednisone (prednisolone), epirubicin, vinblastine, vincristine, dactinomycin (actinomycin), mitomycin C, taxol, L-asparaginase, etoposide, colchicine, deferoxamine mesylate, camptothecin and the like. Furthermore, the antibody may be an immunoconjugate with a radionuclide, toxin and the like.

The agent of the present invention can comprise, in addition to a substance capable of suppressing the expression of a leukemic stem cell marker gene or the activity of a translation product of the gene, an optionally chosen carrier, for example, a pharmaceutically acceptable carrier. Examples of pharmaceutically acceptable carriers include, but are not limited to, excipients such as sucrose, starch, mannitol, sorbitol, lactose, glucose, cellulose, talc, calcium phosphate and calcium carbonate; binders such as cellulose, methylcellulose, hydroxypropylcellulose, polypropylpyrrolidone, gelatin, acacia, polyethylene glycol, sucrose and starch; disintegrants such as starch, carboxymethylcellulose, hydroxypropyl starch, sodium-glycol-starch, sodium hydrogen carbonate, calcium phosphate and calcium citrate; lubricants such as magnesium stearate, Aerosil, talc and sodium lauryl sulfate; flavoring agents such as citric acid, menthol, glycyrrhizin ammonium salt, glycine and orange powder; preservatives such as sodium benzoate, sodium hydrogen sulfite, methyl paraben and propyl paraben; stabilizers such as citric acid, sodium citrate and acetic acid; suspending agents such as methylcellulose, polyvinylpyrrolidone and aluminum stearate; dispersing agents such as surfactants; diluents such as water, physiological saline and orange juice; base waxes such as cacao butter, polyethylene glycol and refined kerosene; and the like.

Preparations suitable for oral administration are liquids prepared by dissolving an effective amount of a substance in a diluent such as water or physiological saline, capsules, sachets or tablets containing an effective amount of a substance in the form of solids or granules, suspensions prepared by suspending an effective amount of a substance in an appropriate dispersant, emulsions prepared by dispersing and emulsifying a solution of an effective amount of a substance in an appropriate dispersant, or powders, granules and the like.

Preparations suitable for parenteral administration (e.g., intravenous injection, subcutaneous injection, intramuscular injection, topical injection and the like) are aqueous and non-aqueous isotonic sterile injectable liquids, which may contain an antioxidant, a buffer solution, a bacteriostatic agent, an isotonizing agent and the like. Aqueous and non-aqueous sterile suspensions can also be mentioned, which may contain a suspending agent, a solubilizer, a thickening agent, a stabilizer, an antiseptic and the like. These preparations can be enclosed in containers such as ampoules and vials for unit dosage or a plurality of dosages. It is also possible to freeze-dry the active ingredient and a pharmaceutically acceptable carrier, and store the preparation in a state that may be dissolved or suspended in an appropriate sterile vehicle just before use.

Although the dosage of the agent varies depending on the activity and choice of active ingredient, the mode of administration (e.g., oral, parenteral), the seriousness of disease, the animal species which is the subject of administration, the drug tolerance, body weight and age of the subject of administration, and the like, and cannot be generalized, it is normally about 0.001 mg to about 5.0 g as the amount of active ingredient per day for an adult.

The subject of administration of the agent is not particularly limited, as far as it is an animal species having a hematopoietic tissue (bone marrow), and possibly contracting acute myeloid leukemia, and it is preferably a mammal, more preferably a human.

### (Method of production)

The present invention also provides a method for producing a sample containing hematopoietic cells for autologous transplantation or allogeneic transplantation for a patient with acute myeloid leukemia. The production method of the present invention comprises,
a) a step of collecting a sample containing hematopoietic cells from the patient or a donor,
b) a step of bringing the collected sample into contact with at least one kind of substance that recognizes a translation product of a leukemic stem cell marker gene, and
c) a step of sorting cells to which the above-described substance has been bound, and obtaining the sample from which leukemic stem cells have been purged. Accordingly, the present invention makes it possible to substantially remove leukemic stem cells from a sample containing hematopoietic cells for autologous transplantation or allogeneic transplantation, and
provide a sample for transplantation without the fear of recurrences.

The leukemic stem cell marker genes are as mentioned above; for the purpose of purging, however, it is preferred to target at least one kind of cell surface marker gene selected from among the following set of genes:
ADFP, ALOX5AP, CACNB4, CD33, CD3D, CD93, CD97, CLEC12A, DOK2, FCER1G, FCGR2A, GPR34, GPR84, HCST, HOMER3, IL2RA, IL2RG, IL3RA, ITGB2, LY86, P2RY5, PTH2R, SUCNR1, TNFRSF4, TYROBP and VNN1.

a) Step of collecting a sample containing hematopoietic cells from a patient with acute myeloid leukemia or a donor Sample collection is normally achieved by bone marrow aspiration or peripheral blood collection. Bone marrow aspiration is performed on the sternum or ilium on the basis of, for example, the method described in S.E. Haynesworth et al., Bone, 13, 81 (1992) and the like. Specifically, the skin surface of the portion for aspirating the bone marrow is disinfected, and topical anesthesia is performed. The subperiosteal region, in particular, is anesthetized sufficiently. The inner cylinder of the puncture needle is removed, a 10 mL syringe containing 5000 units of heparin is attached, and the required amount of bone marrow fluid is quickly aspirated. On average, 10 mL to 20 mL of bone marrow fluid is aspirated. The puncture needle is removed, and astriction is performed for about 10 minutes. The bone marrow fluid acquired is centrifuged at 1,000×g, and bone marrow cells are recovered, after which the bone marrow cells are washed with PBS (Phosphate Buffered Saline). After the washing step is repeated several times, a sample containing hematopoietic cells can be obtained.

In the case of peripheral blood, collection is performed from a vein. Specifically, the skin surface of the portion for peripheral blood collection is disinfected. The inner cylinder of the injection needle is removed, a 10 mL syringe containing 5000 units of heparin is attached, and the required amount of peripheral blood is quickly aspirated. On average, 10 mL to 20 mL of peripheral blood is aspirated. The injection needle is removed, and astriction is performed for about 10 minutes. The peripheral blood acquired is centrifuged at 1,000×g, and peripheral blood cells are recovered, after which the peripheral blood cells are washed with PBS (Phosphate Buffered Saline). After the washing step is repeated several times, a sample containing hematopoietic cells can be obtained.

### b) Step of bringing the collected sample into contact with a substance that recognizes a translation product of at least one kind of leukemic stem cell marker gene

The substances that recognize a translation product of the marker genes for use in this step include antibodies described above, with particular preference given to antibodies against at least one kind of cell surface marker selected from among ADFP, ALOX5AP, CACNB4, CD33, CD3D, CD93, CD97, CLEC12A, DOK2, FCER1G, FCGR2A, GPR34, GPR84, HCST, HOMER3, IL2RA, IL2RG, IL3RA, ITGB2, LY86, P2RY5, PTH2R, SUCNR1, TNFRSF4, TYROBP and VNN1. Preferably, the antibodies are fluorescently labeled, and preferable fluorescent dyes used for the labeling are fluorescent substances commonly used for flow cytometry. Specific examples of fluorescent dyes include FITC (fluorescein isothiocyanate), PE (phycoerythrin), PerCP (peridinin-chlorophyll-protein), PerCP-Cy5.5, PE-Cy5, PE-Cy7, PE-TR (PE-Texas Red), APC (allophycocyanin), APC-Cy7 and the like. Conditions for the contacting are not particularly limited, as far as a contact between the above-mentioned cell surface marker (antigen) and the antibody can be achieved.

### c) Step of sorting cells to which the above-described substance has bound, and obtaining the sample from which leukemic stem cells have been purged

In this step, cell sorting can easily be accomplished by combining with flow cytometry. The sample in contact with a fluorescently labeled antibody is set to a flow cytometer, and the cells bound to the antibody are sorted; leukemic stem cells can be separated from the sample.

The thus-obtained LSC-purged sample can be used for the treatment of AML patients, without the fear of recurrences, as the LSCs have been efficiently eliminated, whereas HSCs have been concentrated escaping elimination.

### [Examples]

The present invention is hereinafter described in detail by means of the following Examples, by which, however, the invention is not limited in any way.

### Human samples

All experiments were conducted with the approval of the Institutional Review Board for Human Research of the RIKEN Research Center for Allergy and Immunology. Leukemia cells derived from AML patients were collected with informed consent in writing. CB (cord blood) derived from healthy donors, along with informed consent in writing, was collected by the Tokyo Cord Blood Bank. BMMNCs (bone marrow mononuclear cells) derived from healthy donors were obtained from Cambrex (Walkerville, MD). BMMNCs and CBMNCs (cord blood mononuclear cells) derived from AML patients were isolated using density gradient centrifugation.

### FACS and flow cytometric analysis

For fluorescence-activated cell sorting (FACS), BMMNC cells from AML patients were labeled with fluorescent dye-coupled mouse anti-hCD3, anti-hCD4, anti-hCD8, anti-hCD34 and anti-hCD38 monoclonal antibodies (BD Biosciences, San Jose, CA), and recipient BMMNC cells were labeled with mouse anti-hCD45, anti-hCD34 and anti-hCD38 monoclonal antibodies (BD Biosciences); the cells were sorted using FACSAria (BD Biosciences). Doublets were eliminated via analyzing FSC/SSC height and FSC/SSC width. After the sorting, the purity of hCD34+hCD38- and hCD34+ cells was higher than 98%. For flow cytometric analysis, BMMNCs of AML patients, recipient peripheral blood or recipient BM was labeled with the above-described fluorescent dye-coupled mouse anti-hCD3, anti-hCD4, anti-hCD8, anti-hCD34 and anti-hCD38 monoclonal antibodies or mouse anti-hCD45, anti-hCD34 and anti-hCD38 monoclonal antibodies.

### Microarray analysis

Total RNA was extracted using TRIzol Reagent (Invitrogen), and the integrity of the RNA was then assessed with Agilent Bioanalyzer. Biotinylated cRNAs were synthesized using Two-Cycle Target Labeling Kit (Affymetrix) for Human Genome U133 plus 2.0 GeneChip (Affymetrix). For Human Gene 1.0ST GeneChip (Affymetrix), a first round of cDNA synthesis and cRNA amplification were performed using MessageAmp Premier RNA Amplification Kit (Applied Biosystems), and a subsequent second round of cDNA synthesis, biotinylation and fragmentation were performed using WT cDNA Synthesis and Terminal Labeling kits (Affymetrix). Hybridization, washing, staining and scanning were performed according to the manufacturers' instruction. Firstly, the microarray data for each platform was separately analyzed using Bioconductor package (http://www.bioconductor.org/). The signal intensities of probe sets on the microarray platforms were normalized with GC-RMA program (Zhijin et al., J. Am. Stat. Assoc., 99, 909-917, 2004). For each platform, the normalized data was analyzed with RankProd program (Hong et al., Bioinformatics, 22, 2825-2827, 2006) to select genes differentially expressed between LSCs and HSCs with the cutoff p value of 0.01 and the false-positive estimation of 0.05%. When a significantly higher level of expression was observed in LSC than in HSC commonly in both the microarray platforms, the gene was selected as a significant candidate LSC marker gene (Fig. 5, Table 1). In addition, the gene IL2RA, which gave a high hit rate for Human Gene 1.0ST GeneChip, and provided favorable results in the protein level analysis, was also selected as a candidate marker gene, since it is expressed in stem cells resistant to anticancer drugs as described below (Table 1). The localization and the biological function of the candidates were annotated based on information from Ingenuity Pathway Analysis Database (Ingenuity Systems) and Gene Ontology Annotation Database (http://www.ebi.ac.uk/GOA/).

### Quantitative PCR (qPCR) analysis

Ten ng of total RNA from HSCs or LSCs was subjected to cDNA amplification using WT-Ovation RNA Amplification System (Nugen). The cDNA products were diluted 1:7.5 in TE, and 1 µl of the dilution products was used per 25 µl of qPCR reaction. The sequences of doubly-labeled fluorescent probes and gene specific primers (Sigma-Aldrich) were listed in Table 3. PCR reactions were performed using LightCycler 480 (Roche Applied Science) with Platinum Quantitative PCR SuperMix-UDG (Invitrogen). The abundance of the respective transcripts was calculated by the standard curve method (Methods, 25, 386-401, 2001). When any of Kruskal-Wallis, Wilcoxon-Mann-Whitney and Student's t-test in Kaleida Graph software package showed P<0.05, it was determined there is a significant difference in the expression level between LSC and HSC.

### Animals

NOD.Cg-Prkdc^{scid}Il2rg^{tmlWjl}/Sz (NOD/SCID/IL2rgγ^{null}) mice were developed at The Jackson Laboratory (Bar Harbor, ME) by backcrossing a complete null mutation at the Il2rg locus onto the NOD.Cg-Prkdc^{scid} (NOD/SCID) strain (Shultz, L.D. et al. Multiple defects in innate and adaptive immunologic function in NOD/LtSz-scid mice. J Immunol 154, 180-191 (1995)). Mice were bred and maintained under defined flora with irradiated food and acidified water at the animal facility of RIKEN and at The Jackson Laboratory according to guidelines established by the Institutional Animal Committees at the respective institutions.

### Heterologous transplantation

Newborn (within 2 days of birth) NOD/SCID/IL2rg^{null} mice received 150 cGy of total body irradiation using a ¹³⁷Cs-source irradiator, followed by intravenous injection of AML cells within two hours. The recipients were subjected to blood sampling from retro-orbital every 3 - 4 weeks, and human AML transplantation chimerism in peripheral blood was assessed.

### Immunofluorescent labeling and imaging

Para-formaldehyde-fixed decalcified paraffin-embedded sections were prepared from a femoral bone of a primary AML transplantation recipient. The primary antibodies used for labeling were a mouse anti-human CD45 monoclonal antibody (DAKO, Denmark) and a rabbit anti-CD32 monoclonal antibody (Abcam, UK). Laser scanning confocal imaging was obtained using Zeiss LSM Exciter and LSM 710 (Carl Zeiss).

### Immunofluorescent labeling and imaging (2)

Para-formaldehyde-fixed decalcified paraffin-embedded sections were prepared from a femoral bone of a recipient of transplantation of primary AML treated with an anticancer agent, and stained with antibodies against DAPI (nuclear staining: blue); various markers (FCGR2A, AK5, DOK2, LRG1, BIK, IL2RA, WT1, SUCNR1: red); and stationary cell markers (green: CD34 (FCGR2A, AK5, DOK2, LRG1, BIK) or Ki67 (IL2RA, WT1, SUCNR1). Laser scanning confocal imaging was obtained using Zeiss LSM Exciter and LSM 710 (Carl Zeiss) (FIG. 7).

### Table 2: List of genes whose transcription product is expressed in larger amounts in AML CD34+CD38- LSCs than in normal CD34+CD38- HSCs

**[Table 2]**

| GeneID | Gene name | Location | Function | Process | Statistics | | | Ratio of median values (LSC/HSC) | Number of LSC samples showing a higher expression than any HSC samples (Maximum: 5) |
|---|---|---|---|---|---|---|---|---|---|
| | | | | | T-test | Wilcoxon-Mann-Whitney | Kruskal-Wallis | | |
| 123 | ADFP | cell membrane | | | 0.011 | 0.032 | 0.027 | 5.5 | 4 |
| 26289 | AK5 | cytoplasm | signaling molecule | | 0.014 | 0.018 | 0.018 | >10000 | 5 |
| 241 | ALOX5AP | cell membrane | | | 0.125 | 0.016 | 0.014 | 33.8 | 5 |
| 93663 | ARHGAP18 | unknown | signaling molecule | | 0.033 | 0.063 | 0.050 | 2.3 | 4 |
| 638 | BIK | cytoplasm | | apoptosis | 0.087 | 0.016 | 0.014 | 129.4 | 5 |
| 785 | CACNB4 | cell membrane | | | 0.009 | 0.016 | 0.014 | 50.4 | 5 |
| 6352 | CCL5 | extracellular space | cytokine | immunity, cell adhesion | 0.018 | 0.016 | 0.014 | 58.4 | 5 |
| 945 | CD33 | cell membrane | signaling molecule | cell adhesion | 0.002 | 0.016 | 0.014 | 8.0 | 5 |
| 915 | CD3D | cell membrane | transmembranous receptor | | 0.086 | 0.015 | 0.011 | >10000 | 5 |
| 22918 | CD93 | cell membrane | | cell adhesion | 0.018 | 0.016 | 0.014 | 27.1 | 5 |
| 976 | CD97 | cell membrane | transmembranous receptor | immunity, cell adhesion | 0.014 | 0.016 | 0.014 | 5.7 | 5 |
| 160364 | CLEC12A | call membrane | | | 0.049 | 0.016 | 0.014 | 180.2 | 5 |
| 1075 | CTSC | cytoplasm | | immunity | <0.001 | 0.016 | 0.014 | 6.6 | 5 |
| 1586 | CYBB | cytoplasm | | immunity | 0.107 | 0.036 | 0.027 | 639.5 | 4 |
| 9046 | DOK2 | cell membrane | signaling molecule | | 0.080 | 0.019 | 0.014 | 31.5 | 5 |
| 2207 | FCER1G | cell membrane | transmembranous receptor | immunity, apoptosis | 0.042 | 0.016 | 0.014 | 24.5 | 4 |
| 2212 | FCGR2A | cell membrane | transmembranous receptor | | 0.347 | 0.016 | 0.014 | 21.1 | 6 |
| 2519 | FUCA2 | extracellular space | | | 0.005 | 0.016 | 0.014 | 2.7 | 5 |
| 2533 | FYB | nucleus | signaling molecule | immunity | 0.031 | 0.018 | 0.013 | 5.4 | 5 |
| 2857 | GPR34 | cell membrane | transmembranous receptor | | 0.085 | 0.016 | 0.014 | 4.3 | 5 |
| 53831 | GPR84 | cell membrane | transmembranous receptor | | 0.259 | 0.016 | 0.014 | 3521.9 | 5 |
| 3055 | HCK | cytoplasm | signaling molecule | | 0.031 | 0.016 | 0.014 | 82.6 | 5 |
| 10870 | HCST | cell membrane | | | <0.001 | 0.016 | 0.014 | 17.3 | 5 |
| 3082 | HGF | extracellular space | growth factor | | 0.034 | 0.191 | 0.142 | 27.2 | 4 |
| 3142 | HLX | nucleus | transcriptional regulation molecule | | 0.003 | 0.016 | 0.014 | 2.9 | 5 |
| 9454 | HOMERS | cell membrane | signaling molecule | | 0.081 | 0.018 | 0.013 | 330.1 | 5 |
| 3561 | IL2RG | cell membrane | transmembranous receptor | immunity | 0.039 | 0.016 | 0.014 | 3.0 | 5 |
| 3563 | IL3RA | cell membrane | transmembranous receptor | | 0.142 | 0.016 | 0.014 | 2.7 | 5 |
| 3689 | ITGB2 | cell membrane | signaling molecule | cell adhesion, apoptosis | 0.016 | 0.016 | 0.014 | 5.6 | 5 |
| 8956 | LGALSI | extracellular space | | apoptosis | 0.011 | 0.016 | 0.014 | 34.5 | 5 |
| 9404 | LPXN | cytoplasm | signaling molecule | call adhesion | 0.008 | 0.016 | 0.014 | 5.2 | 5 |
| 116844 | LRG1 | extracellular space | | | 0.028 | 0.016 | 0.014 | 18.8 | 5 |
| 9450 | LY86 | cell membrane | | immunity, apoptosis | 0.166 | 0.065 | 0.049 | 14.8 | 4 |
| 11320 | MGAT4A | extracellular space | | | 0.031 | 0.063 | 0.050 | 2.6 | 4 |
| 4689 | NCF4 | cytoplasm | | immunity | 0.008 | 0.016 | 0.014 | 5.7 | 5 |
| 10788 | NEK6 | nucleus | signaling molecule | cell cycle, epoptosis | 0.007 | 0.016 | 0.014 | 5.3 | 5 |
| 10161 | P2RY5 | call membrane | transmembranous receptor | | 0.043 | 0.063 | 0.050 | 20.8 | 4 |
| 5152 | PDE9A | cytoplasm | signaling molecule | | 0.140 | 0.016 | 0.014 | 42.0 | 5 |
| 5163 | PDK1 | cytoplasm | signaling molecule | | 0.004 | 0.016 | 0.014 | 11.7 | 5 |
| 5580 | PRKCD | cytoplasm | signaling molecule | | 0.059 | 0.016 | 0.014 | 24.2 | 5 |
| 10942 | PRSS21 | extracellular space | | | 0.023 | 0.191 | 0.142 | 43.8 | 4 |
| 5746 | PTH2R | cell membrane | transmembranous receptor | | 0.133 | 0.019 | 0.014 | 9.3 | 5 |
| 55647 | RAB20 | cytoplasm | signaling molecule | | 0.117 | 0.019 | 0.014 | 157.1 | 6 |
| 4218 | RAB8A | cytoplasm | signaling molecule | | 0.017 | 0.063 | 0.050 | 2.1 | 3 |
| 5877 | RABIF | unknown | signaling molecule | | 0.008 | 0.016 | 0.014 | 8.1 | 5 |
| 6086 | RNASE2 | extracellular space | | | 0.152 | 0.016 | 0.014 | 88.1 | 5 |
| 29015 | SLC43A3 | extracellular space | | | 0.013 | 0.016 | 0.014 | 3.3 | 5 |
| 56670 | SUCNR1 | cell membrane | transmembranous receptor | | 0.075 | 0.082 | 0.027 | 29.9 | 4 |
| 7076 | TIMP1 | extracellular space | | | 0.020 | 0.032 | 0.027 | 3.4 | 4 |
| 7124 | TNF | extracellula- space | cytokine | immunity, apoptosis | 0.325 | 0.016 | 0.014 | 2855.3 | 5 |
| 7298 | TNFRSF4 | cell membrane | transmembranous receptor | immunity | 0.327 | 0.034 | 0.025 | >10000 | 4 |
| 10673 | TNFSF13B | extracellular space | cytokine | immunity | 0.017 | 0.016 | 0.014 | 6.1 | 5 |
| 54957 | TXNL4B | nucleus | | cell cycle | 0.046 | 0.032 | 0.027 | 3.4 | 4 |
| 7805 | TYROBP | cell membrane | signaling molecule | | 0.016 | 0.016 | 0.014 | 14.8 | 5 |
| 8876 | VNN1 | cell membrane | | immunity, apoptosis, cell adhesion | 0.151 | 0.016 | 0.014 | 11.0 | 5 |
| 7490 | WT1 | nucleus | transcriptional regulation molecule | cell cycle | 0.164 | 0.019 | 0.014 | 100.6 | 5 |
| 11130 | ZWINT | nucleus | | cell cycle | 0.078 | 0.032 | 0.027 | 2.2 | 4 |

### Table 3: List of primers, probes and PCR products used in qRT-PCR

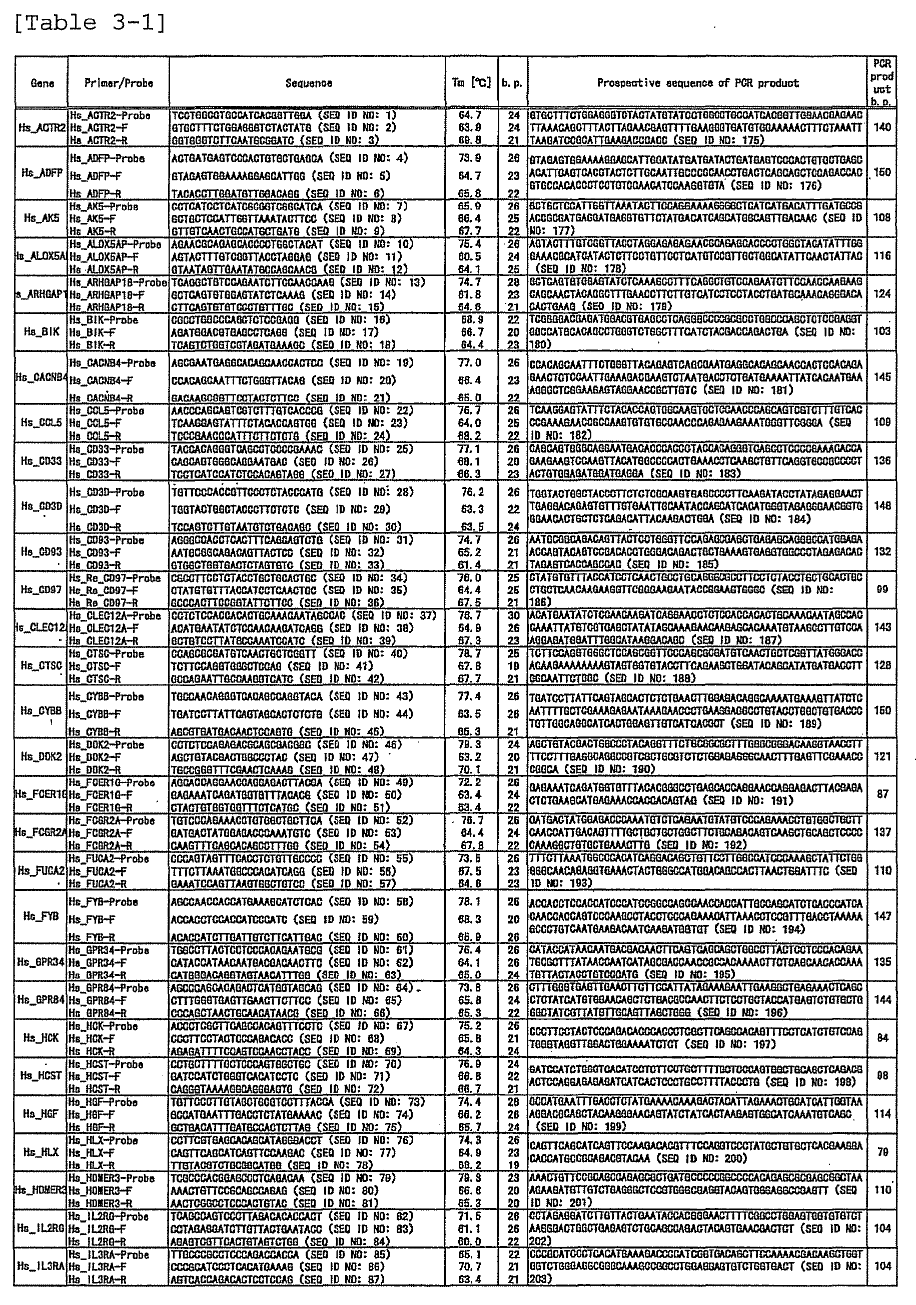

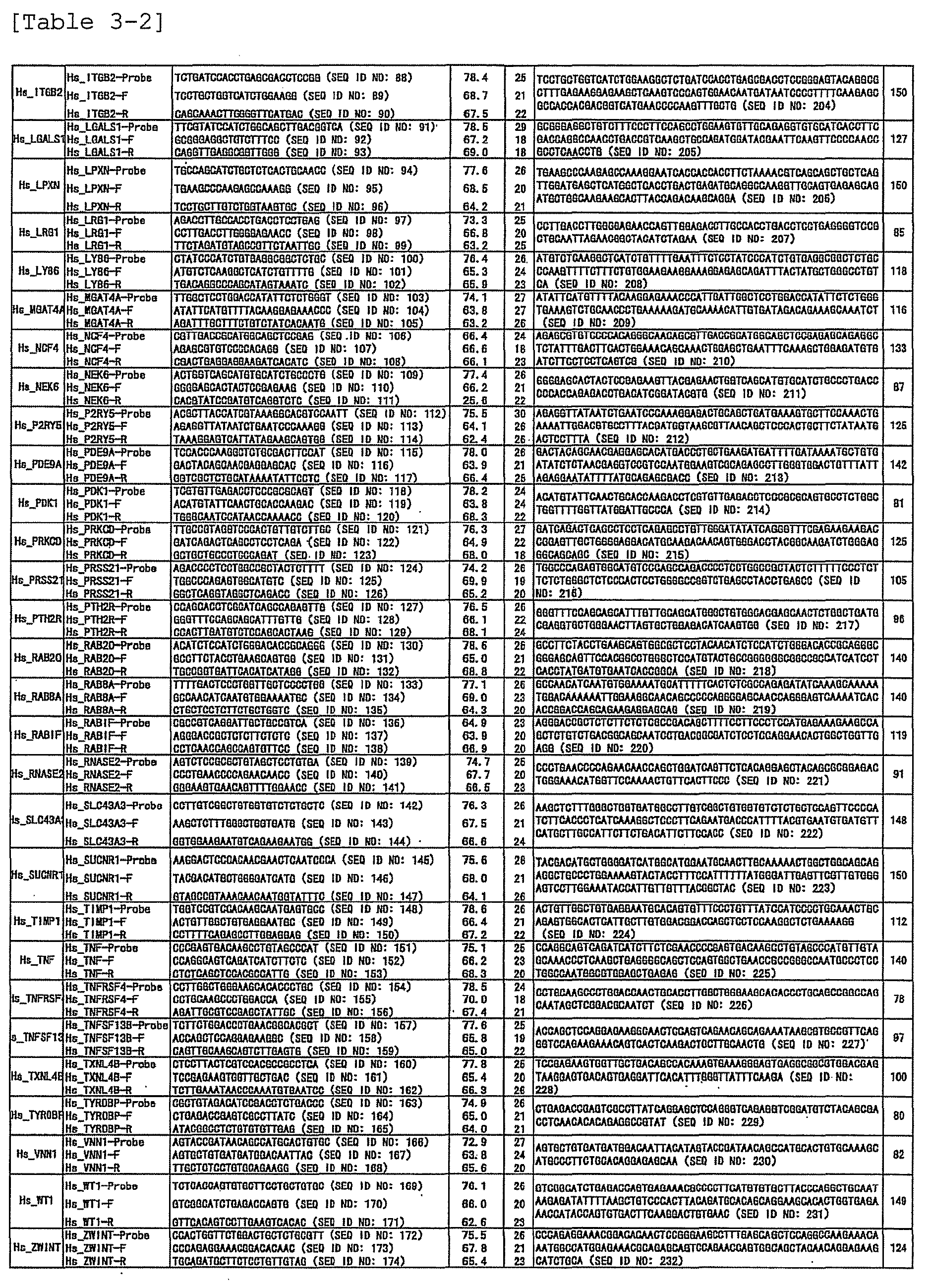

For subsequent qPCR analysis, 121 genes that encode molecules in the following categories were selected from among the 217 genes identified in microarray experiments:
1) Those located on the cell membrane or in extracellular spaces,
2) cytokines, growth factors, transmembranous receptors, protein kinases, phosphatases, transcriptional regulation molecules, and/or signaling molecules, and
3) those involved in immune regulation, cell cycle, apoptosis, and/or cell adhesion.

The list includes 57 genes, the mRNA levels of which were significantly (P<0.05; according to Kruskal-Wallis, Wilcoxon-Mann-Whitney or Student's t-test) higher in LSCs than in HSCs. The columns in Table 2 indicates, in the order from the left column, Entrez Gene ID (Column A), HUGO Gene Symbol (Column B), localization (Column C), molecular function (Column D), biological process (Column E), P values from each statistical test (Columns F-H), ratio of median values of the mRNA levels (Column I), and the number of LSC samples showing a higher expression level than the mRNA levels for the HSC samples (Column J).

The present inventors previously reported that LSCs derived from bone marrow (BM) of AML patient origin and LSCs derived from BM of a mouse receiving transplantation of AML patient BM have similar transcription profiles (Nature Biotechnology, 2007, ibid). Based on this finding, the present inventors performed a comprehensive transcriptome analysis to compare LSCs and normal hematopoietic stem cells (HSCs), using two array platforms: Human Genome U133 plus 2.0 GeneChips (BM derived from 16 AML patients and BM derived from 5 AML transplantation recipient mice were compared with BM derived from 2 healthy donors and cord blood (CB) derived from 5 healthy donors) and Human Gene 1.0ST GeneChips (BM derived from 1 AML patient and BM derived from 5 AML transplantation recipient mice were compared with CB from 1 healthy donor and BM from 4). Since a previous study had revealed that AML stem cells are present exclusively in the CD34+CD38- fraction, >1.2x10⁴ CD34+CD38- cells were recovered with a purity of >98% (FIG. 1). Using the same method, CD34+CD38- HSCs were also purified from normal BM and CB samples (FIG. 1). By intravenously injecting the aforementioned purified HSCs and LSCs to neonatal NOD/SCID/IL2rg KO mice, the onset of AML by LSCs and the lack of reconstitution of normal immunity were confirmed, and long-time transplantation and multi-lineage (T/B/bone marrow) differentiation of HSCs were confirmed (FIG. 1). Not the CD34+CD38+ cells or CD34- cells derived from the AML transplantation recipient mice, but the CD34+CD38- bone marrow cells caused leukemia in secondary recipients. These data suggest that the transplanted CD34+CD38- cells did not come from the HSCs, but retained the nature of the LSCs. To analyze the expression data set obtained, genes that exhibit a significantly higher (p value <0.01, percentage of false positivity <0.05) array signal in LSCs than in HSCs on both the two microarray platforms were extracted using RankProd (Bioinformatics 22, 2825, 2006) mounted on the Bioconductor package. A total of 217 gene candidates met the criteria (FIG. 5, Table 1); further, IL2R was added to make a total of 218 gene candidates.

Next, to demonstrate the expression levels of candidates for separating LSCs and HSCs, quantitative PCR (qPCR) was performed for each candidate gene using LSCs derived from BM of 5 AML patients and HSCs derived from BM of 4 (Table 3). Out of the 217 genes identified, 121 genes that encode molecules in the following categories were selected as candidates best suiting for the development of pharmaceuticals, and subjected to subsequent analysis. The three categories are as follows:
1) those located on the cell membrane or in extracellular spaces,
2) cytokines, growth factors, transmembranous receptors, protein kinases, phosphatases, transcriptional regulation molecules, and/or signaling molecules, and
3) those involved in immune regulation, cell cycle, apoptosis, and/or cell adhesion.

As shown in Table 2, the mRNA contents concerning 57 genes out of the 121 genes were statistically higher in LSCs than in HSCs. Of the 57 genes, 35 genes were identified as excellent LSC markers. The reason was that 1) the median expression levels of these genes were 5 times or higher in LSCs, and that 2) their mRNA contents were higher in all LSC samples tested than in each HSC population tested (FIG. 2).

To confirm the expression of these LSC-specific candidate molecules at the protein level, the quality of monoclonal antibodies and polyclonal antibodies that can be utilized for the 35 candidate molecules, respectively, was verified, and flow cytometric analysis was performed using antibodies proven to be effective and 32 AML patient samples. Through the flow cytometric analysis, the following aspects were examined in each candidate molecule: 1) localization (on cell surfaces or in cells), 2) frequency of positive cells, and 3) expression intensity. Out of the 57 candidate molecules thus assessed, FCGR2A(CD32), ITGB2(CD18), CD93, CD33, CD3D and TNF(TNFa) were found to have the most promising expression level/pattern for LSC-specific markers/targets. In particular, the expression of FCGR2A(CD32) exhibited a strong correlation with LSCs in a significant ratio of the AML patients tested, and this was selected for further functional analysis. In 9 of the 32 AML patients tested, the great majority (>80%) of AML stem cells expressed this antigen (FIG. 3). To confirm that the expression of CD32 correlates exclusively with the function, in vivo NOD/SCID/IL2rg KO transplantation assay was performed using purified LSCs derived from three patients with AML. When purified CD34+CD38-CD32+ and CD34+CD38-CD32- cells were transplanted to sub-lethally irradiated recipients, AML developed exclusively from the CD32+ fraction (FIG. 4). Because any LSC-targeting treatment is thought to be best used along with a commonly used chemotherapeutic agent that is effective in removing non-LSC AML cells, it is important to confirm that the target molecule is continuously expressed even after chemotherapy. Accordingly, the present inventors examined whether the expression of CD32 was maintained after chemotherapy, and confirmed the expression of CD32 in BM, spleens and peripheral blood (PB) of AML transplantation recipient mice after AraC treatment (FIG. 6). Also, CD32-expressing cells were found by immunofluorescent labeling in both the membrane region and central region of bone marrow (FIG. 4). This finding, in view of the previous report by the present inventors that chemotherapy-resistant LSCs are present in BM osteoblast niches, further supports CD32 as a candidate for LSC target therapy (Ishikawa F. et al. Nature Biotechnol 25:1315-1321, 2007 and PCT/JP2008/068892).

Next, the expression of CD32 in normal human HSCs was assessed. In the primary human CB CD34+CD38- population, the frequency of CD32+ cells was 9.8% +/- SD (FIG. 4A). When the expression of CD133 in this fraction was analyzed, CD32+ cells were detected exclusively in the CD34+CD38-CD133- fraction (FIG. 4a). It was found by heterologous transplantation assay that not the CD34+CD38-CD32+ fraction but the CD34+CD38-CD133+CD32- fraction contains HSCs (FIG. 4B). Furthermore, it was suggested by in vitro colony-forming cell (CFC) assay that not CD34+CD38-CD32+ cells but CD34+CD38-CD32- cells have the capability of producing bone marrow-series and erythrocyte-series hematopoietic colonies. The lack of the capability of in vivo long-term hematopoiesis reconstitution in CD32+ normal HSCs suggests the possibility that therapeutic agents targeting CD32 expression cells may help avoid important adverse reactions related to the normal hematopoietic and immune systems without affecting HSCs.

The present inventors first confirmed by neonatal NOD/SCID/IL2rg KO mouse transplantation assay that in AML patient samples lacking the expression of CD32 by LSCs, CD34+CD38- cells possess the LSC function (FIG. 1), and then examined the expression of ITGB2(CD18), CD93, (as well as CD25, CD132, OX41, and CD97), CD33, CD3D and TNFα by flow cytometry. Combination of the antigens CD32, ITGB2, CD93, 97 and 33 enabled good separation of LSCs from normal HSCs in 31 patients out of 47 patients.

The list of LSC-specific genes identified using the two sets of microarrays and quantitative PCR (Table 2) includes genes that are expressed preferentially in bone marrow progeny, but their expression is limited in HSCs. For example, FCGR2A, HCK and NCF4 are highly expressed in mature bone marrow cells and mediate the phagocytosis by immunoconjugates and subsequent superoxide production (Prot Natl Acad Sci USA, 97, 1725; 2000; J Exp Med 191, 669, 2000; Nat Cell Biol, 3, 679, 2001; J Biol Chem 279, 1415, 2004). Meanwhile, CD3D, which is a constituent of the CD3 conjugate, transmits in mature T lymphocytes a T cell receptor signal via the ITAM motif thereof. Therefore, at least a particular ratio of AMLs can develop via abnormal regulation of differentiation in the stem cell stage.

Another feature of the list is the involvement of genes expressed remarkably in cancer cells and leukemia cells. For example, CD33 is a well recognized immunological marker of AML cells, and serves as a target for antibody pharmaceuticals such as gemtuzumab ozogamicin (Leukemia 19: 176, 2005). Furthermore, CD97 has been reported to be accumulated in colorectal cancers that infiltrate lymphatic vessels (Am J Pathol 161, 1657, 2002). Overexpression of these molecules in LSCs suggests that a therapeutic method that targets these molecules may be effective not only on LSCs, but also on mature AML cells. It should be noted that gene products of BIK, HOMER3, WT1 (Genes Chromosomes Cancer 47, 8-20, 2008) and CLEC12A (encoding CLL-1) (Blood 110, 2659-2666, 2007) have been proposed as marker molecules for LSC/AML blasts, and this demonstrates that the findings of the present invention agree with available reports.

By analyzing the expression levels/patterns, the candidate genes were classified into the following sets:
1) a set of genes that encode molecules expressed in a significant ratio of LSCs at the RNA and protein levels, but expressed in only a small ratio of HSCs (or not expressed), and
2) a set of genes expressed at the protein level in LSCs and HSCs, but whose expression intensity as determined by flow cytometry allows separation of LSCs from HSCs.

The gene set 1 includes candidates that specifically target LSCs and do not affect HSCs, for example, promising candidates for the development of therapeutic agents such as antibody pharmaceuticals based on the lack of the aforementioned candidates in normal HSCs. The genes included in the gene set 2 (the most promising candidate is CD33) encode biomarkers having high applicability to ex vivo purging of LSCs for separating LSCs from HSCs and the like against the background of autologous transplantation of hematopoietic stem cells.

As shown in FIG. 7, it was found by identifying the location and the phase in cell cycle by imaging using an antibody against each marker (FCGR2A, AK5, DOK2, LRG1, BIK, IL2RA, WT1 and SUCNR1) and a stationary cell-specific marker, that these molecules are abundantly present in the endosteum (niches), where stem cells exhibiting anticancer agent resistance are present, and are expressed in leukemic stem cells while in the stationary phase of cell cycle. Therefore, targeting these individual marker molecules is thought to be largely contributory to overcoming recurrences of leukemia.

WT1 has been shown to be expressed in a wide variety of tumors, including leukemia. However, whether this molecule is expressed at the level of stem cells, which exhibit recurrences and anticancer agent resistance, has been unknown. The present inventors found that this molecule is expressed in leukemic stem cells that are present in niches and are in the stationary phase of cell cycle, and have shown that the molecule is of significance as a target molecule for killing leukemic stem cells, which have been unable to be killed by conventional chemotherapy and radiotherapy.

Also, peripheral blood was collected from 47 patients with AML in various stages, samples containing hematopoietic cells were prepared, and FCGR2A (CD32a), FCGR2B(CD32b), IL2RA(CD25), ITGB2(CD18) and CD93 positivity rates in leukemic stem cells contained in the samples were examined. The results are shown in Table 4.

**[Table 4]**

| | **n** | **Any marker** | **CD32-a** | **CD32-b** | **CD25** | **CD18** | **CD93** |
|---|---|---|---|---|---|---|---|
| **AML M0** | 2 | 2 | 2 | 0 | 0 | 0 | 0 |
| **% marker positive** | | 100.0 | 100.0 | 0.0 | 0.0 | 0.0 | 0.0 |
| **AML M1** | 7 | 4 | 0 | 2 | 2 | 3 | 0 |
| **% marker positive** | | 57.1 | 0.0 | 28.6 | 28.6 | 42.9 | 0.0 |
| **AML M2** | 14 | 9 | 5 | 4 | 4 | 5 | 1 |
| **% marker positive** | | 64.3 | 35.7 | 28.6 | 28.6 | 35.7 | 7.1 |
| **AML M4** | 4 | 4 | 3 | 1 | 1 | 2 | 1 |
| **% marker positive** | | 100.0 | 75.0 | 25.0 | 25.0 | 50.0 | 25.0 |
| **Other AML** | 3 | 1 | 1 | 0 | 0 | 0 | 0 |
| **% marker positive** | | 33.3 | 33.3 | 0.0 | 0.0 | 0.0 | 0.0 |
| **MDS/AML** | 17 | 11 | 3 | 6 | 9 | 0 | 1 |
| **% marker positive** | | 64.7 | 17.6 | 35.3 | 52.9 | 0.0 | 5.9 |
| **All cases** | 47 | 31 | 14 | 13 | 16 | 10 | 3 |
| **% marker positive** | | 66.0 | 29.8 | 27.7 | 34.0 | 21.3 | 6.4 |

From Table 4, it is seen that by combining 4 kinds of markers FCGR2A(CD32a), IL2RA(CD25), ITGB2(CD18) and CD93, leukemic stem cells can be distinguished at a high rate, and by combining pharmaceuticals that target these 4 kinds of genes, over 60% of leukemia cells can be exterminated.

### Industrial Applicability

By using a leukemic stem cell marker found in the present invention as a molecular target, a therapeutic agent that acts specifically on LSCs that are the source of onset or recurrence of AML can be provided. It is possible to specifically remove LSCs from bone marrow cells of a patient or a donor using a cell sorter such as FACS, with a leukemic stem cell marker found in the present invention as an index. This will increase effectiveness of purging for autologous or allogeneic bone marrow transplantation, and enable to significantly prevent recurrences or the initial onset of acute myeloid leukemia. Furthermore, the presence or absence of LSCs in a collected biological sample or in a living organism can be determined with a leukemic stem cell marker found in the present invention as an index, whereby recurrences or the initial onset of acute myeloid leukemia can also be predicted.

## Claims

1. A test method for predicting the initial onset or a recurrence of acute myeloid leukemia, comprising
(1) a step of measuring the expression level of leukemic stem cell marker genes in a biological sample collected from a subject for a transcription product or translation product of the genes as an analyte, and
(2) a step of comparing the expression levels obtained in the measuring step with a reference value;
wherein the leukemic stem cell marker genes are selected from:
(i) 3 - 35 genes, wherein one of the genes is FCGR2A, and the other genes are selected from the group consisting of: cell membrane- or extracellularly-localized genes consisting of ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4;
(ii) 6 genes consisting of FCGR2A, ITGB2, CD93, CD33, CD3D and TNF;
(iii) 4 genes consisting of FCGR2A, IL2RA, ITGB2 and CD93; or
(iv) 8 genes consisting of FCGR2A, AK5, DOK2, LRG1, BIK, IL2RA, WT1 and SUCNR1;
wherein the reference value for each of the markers is the mean expression value of the marker in CD34+CD38-hematopoietic stem cells for healthy persons or the mean expression value of the marker in CD34+CD38- hematopoietic stem cells for the subject before onset;
and wherein when the expression of two or more leukemic stem cell marker genes in the subject is significantly higher than the reference value, a possible presence of a leukemic stem cell in the collected biological sample or the subject's body is suggested.

2. The test method according to claim 1, wherein the leukemic stem cell marker genes are:
(i') 5 - 35 genes, wherein one of the genes is FCGR2A, and the other genes are selected from the group consisting of: cell membrane- or extracellularly-localized genes consisting of ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4.

3. A production method of a sample containing hematopoietic cells for autologous transplantation or allogeneic transplantation for a patient with acute myeloid leukemia, comprising:
a) a step of collecting a sample containing hematopoietic cells from the patient or a donor;
b) a step of bringing the collected sample into contact with antibodies that recognize a translation product of leukemic stem cell marker genes selected from:
(i) 3 - 35 genes, wherein one of the genes is FCGR2A, and the other genes are selected from the group consisting of: cell membrane- or extracellularly-localized genes consisting of ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP and VNN1; a cell cycle-related gene consisting of NEK6; an apoptosis-related gene consisting of BIK; signaling-related genes consisting of AK5, FYB, HCK, LPXN, PDE9A, PDK1, PRKCD and RAB20; a transcription factor gene consisting of WT1; and other genes consisting of CTSC and NCF4;
(ii) 6 genes consisting of FCGR2A, ITGB2, CD93, CD33, CD3D and TNF;
(iii) 4 genes consisting of FCGR2A, IL2RA, ITGB2 and CD93; or
(iv) 8 genes consisting of FCGR2A, AK5, DOK2, LRG1, BIK, IL2RA, WT1 and SUCNR1; and
c) a step of sorting cells to which the antibodies have bound, and obtaining the sample from which leukemic stem cells have been purged.

## Patentansprüche

1. Testverfahren zur Vorhersage des ersten Einsetzens oder eines Rezidivs von akuter myeloider Leukämie, umfassend:
(1) einen Schritt des Messens des Expressionsniveaus von Leukämie-Stammzell-Markergenen in einer biologischen Probe, die einem Probanden entnommen wurde, bezüglich eines Transkriptionsprodukts oder Translationsprodukts der Gene als Analyt; und
(2) einen Schritt des Vergleichens der in dem Messschritt erhaltenen Expressionsniveaus mit einem Referenzwert;
wobei die Leukämie-Stammzell-Markergene ausgewählt sind aus:
(i) 3 bis 35 Genen, wobei eines der Gene FCGR2A ist und die anderen Gene aus der Gruppe ausgewählt sind, die aus folgenden besteht: in der Zellmembran oder extrazellulär lokalisierten Genen, die aus ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP und VNN1 bestehen; einem mit dem Zellzyklus zusammenhängenden Gen, das aus NEK6 besteht; einem mit Apoptose zusammenhängenden Gen, das aus BIK besteht; mit der Signalgebung zusammenhängenden Genen, die aus AK5, FYB, HCK, LPXN, PDE9A, PDK1, PRKCD und RAB20 bestehen; einem Transkriptionsfaktorgen, das aus WT1 besteht; und anderen Genen, die aus CTSC und NCF4 bestehen;
(ii) 6 Genen, die aus FCGR2A, ITGB2, CD93, CD33, CD3D und TNF bestehen;
(iii) 4 Genen, die aus FCGR2A, IL2RA, ITGB2 und CD93 bestehen; oder
(iv) 8 Genen, die aus FCGR2A, AK5, DOK2, LRG1, BIK, IL2RA, WT1 und SUCNR1 bestehen;
wobei der Referenzwert für jeden der Marker der mittlere Expressionswert des Markers in CD34-positiven, CD38-negativen hämatopoetischen Stammzellen für gesunde Personen oder der mittlere Expressionswert des Markers in CD34-positiven, CD38-negativen hämatopoetischen Stammzellen für den Probanden vor dem Einsetzen ist;
und wobei dann, wenn die Expression von zwei oder mehr Leukämie-Stammzell-Markergenen in dem Probanden signifikant höher ist als der Referenzwert, eine mögliche Anwesenheit einer Leukämie-Stammzelle in der entnommenen biologischen Probe oder dem Körper des Probanden zu vermuten ist.

2. Testverfahren gemäß Anspruch 1, wobei die Leukämie-Stammzell-Markergene folgende sind:
(i') 5 bis 35 Gene, wobei eines der Gene FCGR2A ist und die anderen Gene aus der Gruppe ausgewählt sind, die aus folgenden besteht: in der Zellmembran oder extrazellulär lokalisierten Genen, die aus ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP und VNN1 bestehen; einem mit dem Zellzyklus zusammenhängenden Gen, das aus NEK6 besteht; einem mit Apoptose zusammenhängenden Gen, das aus BIK besteht; mit der Signalgebung zusammenhängenden Genen, die aus AK5, FYB, HCK, LPXN, PDE9A, PDK1, PRKCD und RAB20 bestehen; einem Transkriptionsfaktorgen, das aus WT1 besteht; und anderen Genen, die aus CTSC und NCF4 bestehen.

3. Herstellungsverfahren für eine Probe, die hämatopoetische Zellen für die autologe Transplantation oder allogene Transplantation bei einem Patienten mit akuter myeloider Leukämie enthält, umfassend:
a) einen Schritt des Entnehmens einer Probe, die hämatopoetische Zellen enthält, von dem Patienten oder einem Spender;
b) einen Schritt des In-Kontakt-Bringens der entnommenen Probe mit Antikörpern, die ein Translationsprodukt von Leukämie-Stammzell-Markergenen erkennen, welche ausgewählt sind aus:
(i) 3 bis 35 Genen, wobei eines der Gene FCGR2A ist und die anderen Gene aus der Gruppe ausgewählt sind, die aus folgenden besteht: in der Zellmembran oder extrazellulär lokalisierten Genen, die aus ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP und VNN1 bestehen; einem mit dem Zellzyklus zusammenhängenden Gen, das aus NEK6 besteht; einem mit Apoptose zusammenhängenden Gen, das aus BIK besteht; mit der Signalgebung zusammenhängenden Genen, die aus AK5, FYB, HCK, LPXN, PDE9A, PDK1, PRKCD und RAB20 bestehen; einem Transkriptionsfaktorgen, das aus WT1 besteht; und anderen Genen, die aus CTSC und NCF4 bestehen;
(ii) 6 Genen, die aus FCGR2A, ITGB2, CD93, CD33, CD3D und TNF bestehen;
(iii) 4 Genen, die aus FCGR2A, IL2RA, ITGB2 und CD93 bestehen; oder
(iv) 8 Genen, die aus FCGR2A, AK5, DOK2, LRG1, BIK, IL2RA, WT1 und SUCNR1 bestehen; und
c) einen Schritt des Aussortierens von Zellen, an die die Antikörper gebunden haben, und Erhalten der Probe, aus der Leukämie-Stammzellen entfernt wurden.

## Revendications

1. Méthode de test pour la prédiction de l'apparition initiale ou d'une récurrence d'une leucémie myéloïde aiguë, comprenant
(1) une étape de mesure du taux d'expression de gènes marqueurs des cellules souches leucémiques dans un échantillon biologique prélevé chez un sujet pour un produit de transcription ou un produit de traduction des gènes en tant qu'analyte, et
(2) une étape de comparaison des taux d'expression obtenus dans l'étape de mesure à une valeur de référence ;
dans laquelle les gènes marqueurs des cellules souches leucémiques sont sélectionnés parmi :
(i) 3 à 35 gènes, dans lesquels l'un des gènes est FCGR2A, et les autres gènes sont sélectionnés dans le groupe consistant en : des gènes localisés dans la membrane cellulaire ou au niveau extracellulaire consistant en ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP et VNN1 ; un gène associé au cycle cellulaire consistant en NEK6 ; un gène associé à l'apoptose consistant en BIK ; des gènes associés à la signalisation consistant en AK5, FYB, HCK, LPXN, PDE9A, PDK1, PRKCD et RAB20 ; un gène de facteur de transcription consistant en WT1 ; et d'autres gènes consistant en CTSC et NCF4 ;
(ii) 6 gènes consistant en FCGR2A, ITGB2, CD93, CD33, CD3D et TNF ;
(iii) 4 gènes consistant en FCGR2A, IL2RA, ITGB2 et CD93 ; ou
(iv) 8 gènes consistant en FCGR2A, AK5, DOK2, LRG1, BIK, IL2RA, WT1 et SUCNR1 ;
dans laquelle la valeur de référence pour chacun des marqueurs est la valeur d'expression moyenne du marqueur dans des cellules souches hématopoïétiques CD34+ CD38-pour des personnes saines ou la valeur d'expression moyenne du marqueur dans des cellules souches hématopoïétiques CD34+ CD38- pour le sujet avant l'apparition ;
et dans laquelle lorsque l'expression de deux gènes marqueurs des cellules souches leucémiques ou plus chez le sujet est significativement supérieure à la valeur de référence, une présence possible d'une cellule souche leucémique dans l'échantillon biologique prélevé ou le corps du sujet est suggérée.

2. Méthode de test selon la revendication 1, dans laquelle les gènes marqueurs des cellules souches leucémiques sont :
(i') 5 à 35 gènes, dans lesquels l'un des gènes est FCGR2A, et les autres gènes sont sélectionnés dans le groupe consistant en : des gènes localisés dans la membrane cellulaire ou au niveau extracellulaire consistant en ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP et VNN1 ; un gène associé au cycle cellulaire consistant en NEK6 ; un gène associé à l'apoptose consistant en BIK ; des gènes associés à la signalisation consistant en AK5, FYB, HCK, LPXN, PDE9A, PDK1, PRKCD et RAB20 ; un gène de facteur de transcription consistant en WT1 ; et d'autres gènes consistant en CTSC et NCF4.

3. Méthode de production d'un échantillon contenant des cellules hématopoïétiques pour une transplantation autologue ou une transplantation allogénique pour un patient atteint de leucémie myéloïde aiguë, comprenant :
a) une étape de prélèvement d'un échantillon contenant des cellules hématopoïétiques chez le patient ou un donneur ;
b) une étape de mise en contact de l'échantillon prélevé avec des anticorps qui reconnaissent un produit de traduction des gènes marqueurs des cellules souches leucémiques sélectionnés parmi :
(i) 3 à 35 gènes, dans lesquels l'un des gènes est FCGR2A, et les autres gènes sont sélectionnés dans le groupe consistant en : des gènes localisés dans la membrane cellulaire ou au niveau extracellulaire consistant en ALOX5AP, CACNB4, CCL5, CD33, CD3D, CD93, CD97, CLEC12A, DOK2, GPR84, HCST, HOMER3, ITGB2, LGALS1, LRG1, PTH2R, RNASE2, TNF, TNFSF13B, TYROBP et VNN1 ; un gène associé au cycle cellulaire consistant en NEK6 ; un gène associé à l'apoptose consistant en BIK ; des gènes associés à la signalisation consistant en AK5, FYB, HCK, LPXN, PDE9A, PDK1, PRKCD et RAB20 ; un gène de facteur de transcription consistant en WT1 ; et d'autres gènes consistant en CTSC et NCF4 ;
(ii) 6 gènes consistant en FCGR2A, ITGB2, CD93, CD33, CD3D et TNF ;
(iii) 4 gènes consistant en FCGR2A, IL2RA, ITGB2 et CD93 ; ou
(iv) 8 gènes consistant en FCGR2A, AK5, DOK2, LRG1, BIK, IL2RA, WT1 et SUCNR1 ; et
c) une étape de tri des cellules auxquelles les anticorps se sont liés, et l'obtention de l'échantillon à partir duquel les cellules souches leucémiques ont été purgées.


### SEQUENCE LISTING

<110> RIKEN
<120> Marker for Leukemia Stem Cells
<130> 091532
<150> JP2009-072400
   <151> 2009-03-24
<160> 232
<170> PatentIn version 3.3
<210> 1
   <211> 24
   <212> DNA
   <213> Artificial
<220>
   <223> Probe
<400> 1
   tcctggcctg ccatcacggt tgga 24
<210> 2
   <211> 24
   <212> DNA
   <213> Artificial
<220>
   <223> Primer
<400> 2
   gtgctttctg gagggtctac tatg 24
<210> 3
   <211> 21
   <212> DNA
   <213> Artificial
<220>
   <223> Primer
<400> 3
   ggtgggtctt caatgcggat c 21
<210> 4
   <211> 26
   <212> DNA
   <213> Artificial
<220>
   <223> Probe
<400> 4
   actgatgagt cccactgtgc tgagca 26
<210> 5
   <211> 23
   <212> DNA
   <213> Artificial
<220>
   <223> Primer
<400> 5
   gtagagtgga aaaggagcat tgg 23
<210> 6
   <211> 22
   <212> DNA
   <213> Artificial
<220>
   <223> Primer
<400> 6
   tacaccttgg atgttggaca gg 22
<210> 7
   <211> 26
   <212> DNA
   <213> Artificial
<220>
   <223> Probe
<400> 7
   cctcatcctc atcgcggtcg gcatca 26
<210> 8
   <211> 25
   <212> DNA
   <213> Artificial
<220>
   <223> Primer
<400> 8
   gctgctccat tggttaaata cttcc 25
<210> 9
   <211> 22
   <212> DNA
   <213> Artificial
<220>
   <223> Primer
<400> 9
   gttgtcaact gccatgctga tg 22
<210> 10
   <211> 26
   <212> DNA
   <213> Artificial
<220>
   <223> Probe
<400> 10
   agaacgcaga gcacccctgg ctacat 26
<210> 11
   <211> 24
   <212> DNA
   <213> Artificial
<220>
   <223> Primer
<400> 11
   agtactttgt cggttaccta ggag 24
<210> 12
   <211> 25
   <212> DNA
   <213> Artificial
<220>
   <223> Primer
<400> 12
   gtaatagttg aatatgccag caacg 25
<210> 13
   <211> 28
   <212> DNA
   <213> Artificial
<220>
   <223> Probe
<400> 13
   tcaggctgtc cagaatcttc caaccaag 28
<210> 14
   <211> 23
   <212> DNA
   <213> Artificial
<220>
   <223> Primer
<400> 14
   gctcagtgtg gagtatctca aag 23
<210> 15
   <211> 21
   <212> DNA
   <213> Artificial
<220>
   <223> Primer
<400> 15
   cttcagtgtg tccctgtttg c 21
<210> 16
   <211> 22
   <212> DNA
   <213> Artificial
<220>
   <223> Probe
<400> 16
   cgcctggccc agctctccga gg 22
<210> 17
   <211> 20
   <212> DNA
   <213> Artificial
<220>
   <223> Primer
<400> 17
   agatggacgt gagcctcagg 20
<210> 18
   <211> 23
   <212> DNA
   <213> Artificial
<220>
   <223> Primer
<400> 18
   tcagtctggt cgtagatgaa agc 23
<210> 19
   <211> 26
   <212> DNA
   <213> Artificial
<220>
   <223> Probe
<400> 19
   agcgaatgag gcacagcaac cactcc 26
<210> 20
   <211> 23
   <212> DNA
   <213> Artificial
<220>
   <223> Primer
<400> 20
   ccacagcaat ttctgggtta cag 23
<210> 21
   <211> 22
   <212> DNA
   <213> Artificial
<220>
   <223> Primer
<400> 21
   gacaagcggt tcctactctt cc 22
<210> 22
   <211> 26
   <212> DNA
   <213> Artificial
<220>
   <223> Probe
<400> 22
   aacccagcag tcgtctttgt cacccg 26
<210> 23
   <211> 25
   <212> DNA
   <213> Artificial
<220>
   <223> Primer
<400> 23
   tcaaggagta tttctacacc agtgg 25
<210> 24
   <211> 22
   <212> DNA
   <213> Artificial
<220>
   <223> Primer
<400> 24
   tcccgaaccc atttcttctc tg 22
<210> 25
   <211> 26
   <212> DNA
   <213> Artificial
<220>
   <223> Probe
<400> 25
   taccacaggg tcagcctccc cgaaac 26
<210> 26
   <211> 20
   <212> DNA
   <213> Artificial
<220>
   <223> Primer
<400> 26
   cagcagtggg caggaatgac 20
<210> 27
   <211> 23
   <212> DNA
   <213> Artificial
<220>
   <223> Primer
<400> 27
   tcctcatcca tctccacagt agg 23
<210> 28
   <211> 26
   <212> DNA
   <213> Artificial
<220>
   <223> Probe
<400> 28
   tgttcccacc gttccctcta cccatg 26
<210> 29
   <211> 22
   <212> DNA
   <213> Artificial
<220>
   <223> Primer
<400> 29
   tggtactggc tacccttctc tc 22
<210> 30
   <211> 24
   <212> DNA
   <213> Artificial
<220>
   <223> Primer
<400> 30
   tccagtcttg taatgtctga cagc 24
<210> 31
   <211> 26
   <212> DNA
   <213> Artificial
<220>
   <223> Probe
<400> 31
   agggccacct cactttcagc agtctg 26
<210> 32
   <211> 21
   <212> DNA
   <213> Artificial
<220>
   <223> Primer
<400> 32
   aatgcggcag acagttactc c 21
<210> 33
   <211> 21
   <212> DNA
   <213> Artificial
<220>
   <223> Primer
<400> 33
   gtggctggtg actctagtgt c 21
<210> 34
   <211> 25
   <212> DNA
   <213> Artificial
<220>
   <223> Probe
<400> 34
   cgccttcctc tacctgctgc actgc 25
<210> 35
   <211> 25
   <212> DNA
   <213> Artificial
<220>
   <223> Primer
<400> 35
   ctatgtgttt accatcctca actgc 25
<210> 36
   <211> 21
   <212> DNA
   <213> Artificial
<220>
   <223> Primer
<400> 36
   gcccacttcc ggtattcttc c 21
<210> 37
   <211> 30
   <212> DNA
   <213> Artificial
<220>
   <223> Probe
<400> 37
   cctctccacc acactgcaaa caatagccac 30
<210> 38
   <211> 26
   <212> DNA
   <213> Artificial
<220>
   <223> Primer
<400> 38
   acatgaatat ctccaacaag atcagg 26
<210> 39
   <211> 23
   <212> DNA
   <213> Artificial
<220>
   <223> Primer
<400> 39
   gctgtcctta tgccaaatcc atc 23
<210> 40
   <211> 25
   <212> DNA
   <213> Artificial
<220>
   <223> Probe
<400> 40
   ccagcgcgat gtcaactgct cggtt 25
<210> 41
   <211> 19
   <212> DNA
   <213> Artificial
<220>
   <223> Primer
<400> 41
   tcttccaggt gggctccag 19
<210> 42
   <211> 21
   <212> DNA
   <213> Artificial
<220>
   <223> Primer
<400> 42
   gccagaattg ccaaggtcat c 21
<210> 43
   <211> 26
   <212> DNA
   <213> Artificial
<220>
   <223> Probe
<400> 43
   tgccaacagg gtcacagcca ggtaca 26
<210> 44
   <211> 26
   <212> DNA
   <213> Artificial
<220>
   <223> Primer
<400> 44
   tgatccttat tcagtagcac tctctg 26
<210> 45
   <211> 21
   <212> DNA
   <213> Artificial
<220>
   <223> Primer
<400> 45
   agcgtgatga caactccagt g 21
<210> 46
   <211> 24
   <212> DNA
   <213> Artificial
<220>
   <223> Probe
<400> 46
   cctctccaga gacgcagcga cggc 24
<210> 47
   <211> 20
   <212> DNA
   <213> Artificial
<220>
   <223> Primer
<400> 47
   agctgtacga ctggccctac 20
<210> 48
   <211> 21
   <212> DNA
   <213> Artificial
<220>
   <223> Primer
<400> 48
   tgccgggttt cgaactcaaa g 21
<210> 49
   <211> 26
   <212> DNA
   <213> Artificial
<220>
   <223> Probe
<400> 49
   agcaccagga accaggagac ttacga 26
<210> 50
   <211> 24
   <212> DNA
   <213> Artificial
<220>
   <223> Primer
<400> 50
   gagaaatcag atggtgttta cacg 24
<210> 51
   <211> 22
   <212> DNA
   <213> Artificial
<220>
   <223> Primer
<400> 51
   ctactgtggt ggtttctcat gc 22
<210> 52
   <211> 26
   <212> DNA
   <213> Artificial
<220>
   <223> Probe
<400> 52
   tgtcccagaa acctgtggct gcttca 26
<210> 53
   <211> 24
   <212> DNA
   <213> Artificial
<220>
   <223> Primer
<400> 53
   gatgactatg gagacccaaa tgtc 24
<210> 54
   <211> 22
   <212> DNA
   <213> Artificial
<220>
   <223> Primer
<400> 54
   caagtttcag cacagccttt gg 22
<210> 55
   <211> 26
   <212> DNA
   <213> Artificial
<220>
   <223> Probe
<400> 55
   cccagtagtt tcacctctgt tgcccc 26
<210> 56
   <211> 23
   <212> DNA
   <213> Artificial
<220>
   <223> Primer
<400> 56
   tttcttaaat ggcccacatc agg 23
<210> 57
   <211> 23
   <212> DNA
   <213> Artificial
<220>
   <223> Primer
<400> 57
   gaaatccagt taagtggctg tcc 23
<210> 58
   <211> 26
   <212> DNA
   <213> Artificial
<220>
   <223> Probe
<400> 58
   agccaaccac catgaaagca tctcac 26
<210> 59
   <211> 20
   <212> DNA
   <213> Artificial
<220>
   <223> Primer
<400> 59
   accacctcca ccatcccatc 20
<210> 60
   <211> 26
   <212> DNA
   <213> Artificial
<220>
   <223> Primer
<400> 60
   acaccatctt gattgtcttc attgac 26
<210> 61
   <211> 26
   <212> DNA
   <213> Artificial
<220>
   <223> Probe
<400> 61
   tggccttact cctcccacag aatgcg 26
<210> 62
   <211> 26
   <212> DNA
   <213> Artificial
<220>
   <223> Primer
<400> 62
   cataccataa caatgacgac aacttc 26
<210> 63
   <211> 24
   <212> DNA
   <213> Artificial
<220>
   <223> Primer
<400> 63
   catgggacag gtagtaacat ttgg 24
<210> 64
   <211> 26
   <212> DNA
   <213> Artificial
<220>
   <223> Probe
<400> 64
   agcccagcac agactcatgg tagcag 26
<210> 65
   <211> 24
   <212> DNA
   <213> Artificial
<220>
   <223> Primer
<400> 65
   ctttgggtga gttgaacttc ttcc 24
<210> 66
   <211> 22
   <212> DNA
   <213> Artificial
<220>
   <223> Primer
<400> 66
   cccagctaac tgcaacataa cg 22
<210> 67
   <211> 26
   <212> DNA
   <213> Artificial
<220>
   <223> Probe
<400> 67
   accctcgctt cagccacagt ttcctc 26
<210> 68
   <211> 21
   <212> DNA
   <213> Artificial
<220>
   <223> Primer
<400> 68
   cccttcctac tcccagacac c 21
<210> 69
   <211> 24
   <212> DNA
   <213> Artificial
<220>
   <223> Primer
<400> 69
   agagattttc cagtccaacc tacc 24
<210> 70
   <211> 24
   <212> DNA
   <213> Artificial
<220>
   <223> Probe
<400> 70
   cctgcttttg ctcccagtgg ctgc 24
<210> 71
   <211> 22
   <212> DNA
   <213> Artificial
<220>
   <223> Primer
<400> 71
   gatccatctg ggtcacatcc tc 22
<210> 72
   <211> 21
   <212> DNA
   <213> Artificial
<220>
   <223> Primer
<400> 72
   cagggtaaaa ggcagggagt g 21
<210> 73
   <211> 28
   <212> DNA
   <213> Artificial
<220>
   <223> Probe
<400> 73
   tgttcccttg tagctgcgtc ctttacca 28
<210> 74
   <211> 26
   <212> DNA
   <213> Artificial
<220>
   <223> Primer
<400> 74
   gccatgaatt tgacctctat gaaaac 26
<210> 75
   <211> 24
   <212> DNA
   <213> Artificial
<220>
   <223> Primer
<400> 75
   gctgacattt gatgccactc ttag 24
<210> 76
   <211> 26
   <212> DNA
   <213> Artificial
<220>
   <223> Probe
<400> 76
   ccttcgtgag cacagcatag ggacct 26
<210> 77
   <211> 23
   <212> DNA
   <213> Artificial
<220>
   <223> Primer
<400> 77
   cagttcagca tcagttccaa gac 23
<210> 78
   <211> 19
   <212> DNA
   <213> Artificial
<220>
   <223> Primer
<400> 78
   ttgtacgtct gcggcatgg 19
<210> 79
   <211> 23
   <212> DNA
   <213> Artificial
<220>
   <223> Probe
<400> 79
   tcgcccacgg agccctcaga caa 23
<210> 80
   <211> 20
   <212> DNA
   <213> Artificial
<220>
   <223> Primer
<400> 80
   aaactgttcc gcagccagag 20
<210> 81
   <211> 20
   <212> DNA
   <213> Artificial
<220>
   <223> Primer
<400> 81
   aactcggcct cccactgtac 20
<210> 82
   <211> 26
   <212> DNA
   <213> Artificial
<220>
   <223> Probe
<400> 82
   tcagccagtc ccttagacac accact 26
<210> 83
   <211> 26
   <212> DNA
   <213> Artificial
<220>
   <223> Primer
<400> 83
   cctagaggat cttgttactg aatacc 26
<210> 84
   <211> 22
   <212> DNA
   <213> Artificial
<220>
   <223> Primer
<400> 84
   agagtcgttc actgtagtct gg 22
<210> 85
   <211> 22
   <212> DNA
   <213> Artificial
<220>
   <223> Probe
<400> 85
   ttgcccgcct cccagaccac ca 22
<210> 86
   <211> 21
   <212> DNA
   <213> Artificial
<220>
   <223> Primer
<400> 86
   cccgcatccc tcacatgaaa g 21
<210> 87
   <211> 21
   <212> DNA
   <213> Artificial
<220>
   <223> Primer
<400> 87
   agtcaccaga cactcctcca g 21
<210> 88
   <211> 25
   <212> DNA
   <213> Artificial
<220>
   <223> Probe
<400> 88
   tctgatccac ctgagcgacc tccgg 25
<210> 89
   <211> 21
   <212> DNA
   <213> Artificial
<220>
   <223> Primer
<400> 89
   tcctgctggt catctggaag g 21
<210> 90
   <211> 22
   <212> DNA
   <213> Artificial
<220>
   <223> Primer
<400> 90
   cagcaaactt ggggttcatg ac 22
<210> 91
   <211> 29
   <212> DNA
   <213> Artificial
<220>
   <223> Probe
<400> 91
   ttcgtatcca tctggcagct tgacggtca 29
<210> 92
   <211> 18
   <212> DNA
   <213> Artificial
<220>
   <223> Primer
<400> 92
   gcgggaggct gtctttcc 18
<210> 93
   <211> 18
   <212> DNA
   <213> Artificial
<220>
   <223> Primer
<400> 93
   caggttgagg cggttggg 18
<210> 94
   <211> 26
   <212> DNA
   <213> Artificial
<220>
   <223> Probe
<400> 94
   tgccagcatc tgctctcact gcaacc 26
<210> 95
   <211> 20
   <212> DNA
   <213> Artificial
<220>
   <223> Primer
<400> 95
   tgaagcccaa gagccaaagg 20
<210> 96
   <211> 21
   <212> DNA
   <213> Artificial
<220>
   <223> Primer
<400> 96
   tcctgcttgt ctggtaagtg c 21
<210> 97
   <211> 25
   <212> DNA
   <213> Artificial
<220>
   <223> Probe
<400> 97
   agaccttgcc acctgacctc ctgag 25
<210> 98
   <211> 20
   <212> DNA
   <213> Artificial
<220>
   <223> Primer
<400> 98
   ccttgacctt ggggagaacc 20
<210> 99
   <211> 25
   <212> DNA
   <213> Artificial
<220>
   <223> Primer
<400> 99
   ttctagatgt agccgttcta attgc 25
<210> 100
   <211> 26
   <212> DNA
   <213> Artificial
<220>
   <223> Probe
<400> 100
   ctatcccatc tgtgaggcgg ctctgc 26
<210> 101
   <211> 24
   <212> DNA
   <213> Artificial
<220>
   <223> Primer
<400> 101
   atgtctcaag gctcatctgt tttg 24
<210> 102
   <211> 23
   <212> DNA
   <213> Artificial
<220>
   <223> Primer
<400> 102
   tgacaggccc agcatagtaa atc 23
<210> 103
   <211> 27
   <212> DNA
   <213> Artificial
<220>
   <223> Probe
<400> 103
   ttggctcctg gaccatattc tctgggt 27
<210> 104
   <211> 27
   <212> DNA
   <213> Artificial
<220>
   <223> Primer
<400> 104
   atattcatgt tttacaagga gaaaccc 27
<210> 105
   <211> 26
   <212> DNA
   <213> Artificial
<220>
   <223> Primer
<400> 105
   agatttgctt tctgtctatc acaatg 26
<210> 106
   <211> 24
   <212> DNA
   <213> Artificial
<220>
   <223> Probe
<400> 106
   cgttgaccgc atggcagctc cgag 24
<210> 107
   <211> 18
   <212> DNA
   <213> Artificial
<220>
   <223> Primer
<400> 107
   agagcgtgtc cccacagg 18
<210> 108
   <211> 23
   <212> DNA
   <213> Artificial
<220>
   <223> Primer
<400> 108
   cgactgagga ggaagatcac atc 23
<210> 109
   <211> 26
   <212> DNA
   <213> Artificial
<220>
   <223> Probe
<400> 109
   actggtcagc atgtgcatct gccctg 26
<210> 110
   <211> 21
   <212> DNA
   <213> Artificial
<220>
   <223> Primer
<400> 110
   ggggagcact actccgagaa g 21
<210> 111
   <211> 22
   <212> DNA
   <213> Artificial
<220>
   <223> Primer
<400> 111
   cacgtatccg atgtcaggtc tc 22
<210> 112
   <211> 30
   <212> DNA
   <213> Artificial
<220>
   <223> Probe
<400> 112
   acgcttacca tcgtaaaggc acgtccaatt 30
<210> 113
   <211> 26
   <212> DNA
   <213> Artificial
<220>
   <223> Primer
<400> 113
   agaggttata atctgaatcc caaagg 26
<210> 114
   <211> 26
   <212> DNA
   <213> Artificial
<220>
   <223> Primer
<400> 114
   taaaggagtc attatagaag cagtgg 26
<210> 115
   <211> 26
   <212> DNA
   <213> Artificial
<220>
   <223> Probe
<400> 115
   tccacccaag gctctgcgac ttccat 26
<210> 116
   <211> 21
   <212> DNA
   <213> Artificial
<220>
   <223> Primer
<400> 116
   gactacagca acgaggagca c 21
<210> 117
   <211> 25
   <212> DNA
   <213> Artificial
<220>
   <223> Primer
<400> 117
   ggtcgctctg cataaaatat tcctc 25
<210> 118
   <211> 24
   <212> DNA
   <213> Artificial
<220>
   <223> Probe
<400> 118
   tcgtgttgag acctcccgcg cagt 24
<210> 119
   <211> 24
   <212> DNA
   <213> Artificial
<220>
   <223> Primer
<400> 119
   acatgtattc aactgcacca agac 24
<210> 120
   <211> 22
   <212> DNA
   <213> Artificial
<220>
   <223> Primer
<400> 120
   tgggcaatcc ataaccaaaa cc 22
<210> 121
   <211> 27
   <212> DNA
   <213> Artificial
<220>
   <223> Probe
<400> 121
   ttgccgtagg tcccactgtt gtcttgc 27
<210> 122
   <211> 22
   <212> DNA
   <213> Artificial
<220>
   <223> Primer
<400> 122
   gatcagactc agcctcctca ga 22
<210> 123
   <211> 18
   <212> DNA
   <213> Artificial
<220>
   <223> Primer
<400> 123
   gctgctgccc tcccagat 18
<210> 124
   <211> 26
   <212> DNA
   <213> Artificial
<220>
   <223> Probe
<400> 124
   agacccctcc tggccgctac tctttt 26
<210> 125
   <211> 19
   <212> DNA
   <213> Artificial
<220>
   <223> Primer
<400> 125
   tggcccagag tggcatgtc 19
<210> 126
   <211> 20
   <212> DNA
   <213> Artificial
<220>
   <223> Primer
<400> 126
   ggctcaggta ggctcagacc 20
<210> 127
   <211> 26
   <212> DNA
   <213> Artificial
<220>
   <223> Probe
<400> 127
   ccagcacctc gcatcagcca gagttg 26
<210> 128
   <211> 22
   <212> DNA
   <213> Artificial
<220>
   <223> Primer
<400> 128
   gggtttccag cagcatttgt tg 22
<210> 129
   <211> 24
   <212> DNA
   <213> Artificial
<220>
   <223> Primer
<400> 129
   ccacttgatg tctccagcac taag 24
<210> 130
   <211> 26
   <212> DNA
   <213> Artificial
<220>
   <223> Probe
<400> 130
   acatctccat ctgggacacc gcaggg 26
<210> 131
   <211> 21
   <212> DNA
   <213> Artificial
<220>
   <223> Primer
<400> 131
   gccttctacc tgaagcagtg g 21
<210> 132
   <211> 22
   <212> DNA
   <213> Artificial
<220>
   <223> Primer
<400> 132
   tgccggtgat tcacatcata gg 22
<210> 133
   <211> 26
   <212> DNA
   <213> Artificial
<220>
   <223> Probe
<400> 133
   ttttgactcc ctggttgctc ccctgg 26
<210> 134
   <211> 23
   <212> DNA
   <213> Artificial
<220>
   <223> Primer
<400> 134
   gccaacatca atgtggaaaa tgc 23
<210> 135
   <211> 20
   <212> DNA
   <213> Artificial
<220>
   <223> Primer
<400> 135
   ctgctcctct tctgctggtc 20
<210> 136
   <211> 23
   <212> DNA
   <213> Artificial
<220>
   <223> Probe
<400> 136
   cgccgtcagg attgctgccg tca 23
<210> 137
   <211> 20
   <212> DNA
   <213> Artificial
<220>
   <223> Primer
<400> 137
   agggaccgct ctcttctctc 20
<210> 138
   <211> 20
   <212> DNA
   <213> Artificial
<220>
   <223> Primer
<400> 138
   cctcaaccag ccagtgttcc 20
<210> 139
   <211> 25
   <212> DNA
   <213> Artificial
<220>
   <223> Probe
<400> 139
   agtctccgcg ctgtagctcc tgtga 25
<210> 140
   <211> 20
   <212> DNA
   <213> Artificial
<220>
   <223> Primer
<400> 140
   ccctgaaccc cagaacaacc 20
<210> 141
   <211> 23
   <212> DNA
   <213> Artificial
<220>
   <223> Primer
<400> 141
   gggaagtgaa cagttttgga acc 23
<210> 142
   <211> 26
   <212> DNA
   <213> Artificial
<220>
   <223> Probe
<400> 142
   ccttgtcggc tgtggtgtct ctgctc 26
<210> 143
   <211> 21
   <212> DNA
   <213> Artificial
<220>
   <223> Primer
<400> 143
   aagctctttg ggctggtgat g 21
<210> 144
   <211> 24
   <212> DNA
   <213> Artificial
<220>
   <223> Primer
<400> 144
   ggtggaagaa tgtcagaaga atgg 24
<210> 145
   <211> 28
   <212> DNA
   <213> Artificial
<220>
   <223> Probe
<400> 145
   aaggactccc acaacgaact caatccca 28
<210> 146
   <211> 21
   <212> DNA
   <213> Artificial
<220>
   <223> Primer
<400> 146
   tacgacatgc tggggatcat g 21
<210> 147
   <211> 26
   <212> DNA
   <213> Artificial
<220>
   <223> Primer
<400> 147
   gtagccgtaa acaacaatgg tatttc 26
<210> 148
   <211> 26
   <212> DNA
   <213> Artificial
<220>
   <223> Probe
<400> 148
   tggtccgtcc acaagcaatg agtgcc 26
<210> 149
   <211> 21
   <212> DNA
   <213> Artificial
<220>
   <223> Primer
<400> 149
   actgttggct gtgaggaatg c 21
<210> 150
   <211> 22
   <212> DNA
   <213> Artificial
<220>
   <223> Primer
<400> 150
   ccttttcaga gccttggagg ag 22
<210> 151
   <211> 25
   <212> DNA
   <213> Artificial
<220>
   <223> Probe
<400> 151
   cccgagtgac aagcctgtag cccat 25
<210> 152
   <211> 23
   <212> DNA
   <213> Artificial
<220>
   <223> Primer
<400> 152
   ccaggcagtc agatcatctt ctc 23
<210> 153
   <211> 20
   <212> DNA
   <213> Artificial
<220>
   <223> Primer
<400> 153
   ctctcagctc cacgccattg 20
<210> 154
   <211> 24
   <212> DNA
   <213> Artificial
<220>
   <223> Probe
<400> 154
   ccttggctgg gaagcacacc ctgc 24
<210> 155
   <211> 18
   <212> DNA
   <213> Artificial
<220>
   <223> Primer
<400> 155
   cctgcaagcc ctggacca 18
<210> 156
   <211> 21
   <212> DNA
   <213> Artificial
<220>
   <223> Primer
<400> 156
   agattgcgtc cgagctattg c 21
<210> 157
   <211> 25
   <212> DNA
   <213> Artificial
<220>
   <223> Probe
<400> 157
   tcttctggac cctgaacggc acgct 25
<210> 158
   <211> 19
   <212> DNA
   <213> Artificial
<220>
   <223> Primer
<400> 158
   accagctcca ggagaaggc 19
<210> 159
   <211> 22
   <212> DNA
   <213> Artificial
<220>
   <223> Primer
<400> 159
   cagttgcaag cagtcttgag tg 22
<210> 160
   <211> 25
   <212> DNA
   <213> Artificial
<220>
   <223> Probe
<400> 160
   ctccttactc gtccacgccg cctca 25
<210> 161
   <211> 20
   <212> DNA
   <213> Artificial
<220>
   <223> Primer
<400> 161
   tccgagaagt ggttgctgac 20
<210> 162
   <211> 26
   <212> DNA
   <213> Artificial
<220>
   <223> Primer
<400> 162
   tcttgaaata acccaaatgt gaatcc 26
<210> 163
   <211> 26
   <212> DNA
   <213> Artificial
<220>
   <223> Probe
<400> 163
   cgctgtagac atccgacctc tgaccc 26
<210> 164
   <211> 21
   <212> DNA
   <213> Artificial
<220>
   <223> Primer
<400> 164
   ctgagaccga gtcgccttat c 21
<210> 165
   <211> 21
   <212> DNA
   <213> Artificial
<220>
   <223> Primer
<400> 165
   atacggcctc tgtgtgttga g 21
<210> 166
   <211> 27
   <212> DNA
   <213> Artificial
<220>
   <223> Probe
<400> 166
   agtaccgata acagccatgc actgtgc 27
<210> 167
   <211> 24
   <212> DNA
   <213> Artificial
<220>
   <223> Primer
<400> 167
   agtgctgtga tgatggacaa ttac 24
<210> 168
   <211> 20
   <212> DNA
   <213> Artificial
<220>
   <223> Primer
<400> 168
   ttgctctcct gtgcagaagg 20
<210> 169
   <211> 26
   <212> DNA
   <213> Artificial
<220>
   <223> Probe
<400> 169
   tctcaccagt gtgcttcctg ctgtgc 26
<210> 170
   <211> 20
   <212> DNA
   <213> Artificial
<220>
   <223> Primer
<400> 170
   gtcggcatct gagaccagtg 20
<210> 171
   <211> 23
   <212> DNA
   <213> Artificial
<220>
   <223> Primer
<400> 171
   gttcacagtc cttgaagtca cac 23
<210> 172
   <211> 26
   <212> DNA
   <213> Artificial
<220>
   <223> Probe
<400> 172
   ccactggttc tggactgctc tgcgtt 26
<210> 173
   <211> 21
   <212> DNA
   <213> Artificial
<220>
   <223> Primer
<400> 173
   cccagaggaa acggacacaa c 21
<210> 174
   <211> 23
   <212> DNA
   <213> Artificial
<220>
   <223> Primer
<400> 174
   tgcagatgct tctcctgttg tag 23
<210> 175
   <211> 140
   <212> DNA
   <213> Artificial
<220>
   <223> PCR product
<400> 175
   gtgctttctg gagggtctac tatgtatcct ggcctgccat cacggttgga acgagaactt     60
   aaacagcttt acttagaacg agttttgaag ggtgatgtgg aaaaactttc taaatttaag    120
   atccgcattg aagacccacc                                                140
<210> 176
   <211> 150
   <212> DNA
   <213> Artificial
<220>
   <223> PCR product
<400> 176
   gtagagtgga aaaggagcat tggatatgat gatactgatg agtcccactg tgctgagcac     60
   attgagtcac gtactcttgc aattgcccgc aacctgactc agcagctcca gaccacgtgc    120
   cacaccctcc tgtccaacat ccaaggtgta                                      150
<210> 177
   <211> 108
   <212> DNA
   <213> Artificial
<220>
   <223> PCR product
<400> 177
   gctgctccat tggttaaata cttccaggaa aaggggctca tcatgacatt tgatgccgac     60
   cgcgatgagg atgaggtgtt ctatgacatc agcatggcag ttgacaac                  108
<210> 178
   <211> 116
   <212> DNA
   <213> Artificial
<220>
   <223> PCR product
<400> 178
   agtactttgt cggttaccta ggagagagaa cgcagagcac ccctggctac atatttggga 60
   aacgcatcat actcttcctg ttcctcatgt ccgttgctgg catattcaac tattac 116
<210> 179
   <211> 124
   <212> DNA
   <213> Artificial
<220>
   <223> PCR product
<400> 179
   gctcagtgtg gagtatctca aagcctttca ggctgtccag aatcttccaa ccaagaagca 60
   gcaactacag gctttgaacc ttcttgtcat cctcctacct gatgcaaaca gggacacact 120
   gaag 124
<210> 180
   <211> 103
   <212> DNA
   <213> Artificial
<220>
   <223> PCR product
<400> 180
   tcggggacga gatggacgtg agcctcaggg ccccgcgcct ggcccagctc tccgaggtgg 60
   ccatgcacag cctgggtctg gctttcatct acgaccagac tga 103
<210> 181
   <211> 145
   <212> DNA
   <213> Artificial
<220>
   <223> PCR product
<400> 181
   ccacagcaat ttctgggtta cagagtcagc gaatgaggca cagcaaccac tccacagaga 60
   actctccaat tgaaagacga agtctaatga cctctgatga aaattatcac aatgaaaggg 120
   ctcggaagag taggaaccgc ttgtc 145
<210> 182
   <211> 109
   <212> DNA
   <213> Artificial
<220>
   <223> PCR product
<400> 182
   tcaaggagta tttctacacc agtggcaagt gctccaaccc agcagtcgtc tttgtcaccc      60
   gaaagaaccg ccaagtgtgt gccaacccag agaagaaatg ggttcggga                 109
<210> 183
   <211> 136
   <212> DNA
   <213> Artificial
<220>
   <223> PCR product
<400> 183
   cagcagtggg caggaatgac acccacccta ccacagggtc agcctccccg aaacaccaga      60
   agaagtccaa gttacatggc cccactgaaa cctcaagctg ttcaggtgcc gcccctactg     120
   tggagatgga tgagga                                                     136
<210> 184
   <211> 148
   <212> DNA
   <213> Artificial
<220>
   <223> PCR product
<400> 184
   tggtactggc tacccttctc tcgcaagtga gccccttcaa gatacctata gaggaacttg      60
   aggacagagt gtttgtgaat tgcaatacca gcatcacatg ggtagaggga acggtgggaa     120
   cactgctctc agacattaca agactgga                                        148
<210> 185
   <211> 132
   <212> DNA
   <213> Artificial
<220>
   <223> PCR product
<400> 185
   aatgcggcag acagttactc ctgggttcca gagcgagctg agagcagggc catggagaac      60
   cagtacagtc cgacacctgg gacagactgc tgaaagtgag gtggccctag agacactaga     120
   gtcaccagcc ac                                                         132
<210> 186
   <211> 99
   <212> DNA
   <213> Artificial
<220>
   <223> PCR product
<400> 186
   ctatgtgttt accatcctca actgcctgca gggcgccttc ctctacctgc tgcactgcct      60
   gctcaacaag aaggttcggg aagaataccg gaagtgggc                             99
<210> 187
   <211> 143
   <212> DNA
   <213> Artificial
<220>
   <223> PCR product
<400> 187
   acatgaatat ctccaacaag atcaggaacc tctccaccac actgcaaaca atagccacca      60
   aattatgtcg tgagctatat agcaaagaac aagagcacaa atgtaagcct tgtccaagga     120
   gatggatttg gcataaggac agc                                             143
<210> 188
   <211> 128
   <212> DNA
   <213> Artificial
<220>
   <223> PCR product
<400> 188
   tcttccaggt gggctccagc ggttcccagc gcgatgtcaa ctgctcggtt atgggaccac      60
   aagaaaaaaa agtagtggtg taccttcaga agctggatac agcatatgat gaccttggca     120
   attctggc                                                              128
<210> 189
   <211> 150
   <212> DNA
   <213> Artificial
<220>
   <223> PCR product
<400> 189
   tgatccttat tcagtagcac tctctgaact tggagacagg caaaatgaaa gttatctcaa 60
   ttttgctcga aagagaataa agaaccctga aggaggcctg tacctggctg tgaccctgtt 120
   ggcaggcatc actggagttg tcatcacgct 150
<210> 190
   <211> 121
   <212> DNA
   <213> Artificial
<220>
   <223> PCR product
<400> 190
   agctgtacga ctggccctac aggtttctgc ggcgctttgg gcgggacaag gtaacctttt 60
   cctttgaggc aggccgtcgc tgcgtctctg gagagggcaa ctttgagttc gaaacccggc 120
   a 121
<210> 191
   <211> 87
   <212> DNA
   <213> Artificial
<220>
   <223> PCR product
<400> 191
   gagaaatcag atggtgttta cacgggcctg agcaccagga accaggagac ttacgagact 60
   ctgaagcatg agaaaccacc acagtag 87
<210> 192
   <211> 137
   <212> DNA
   <213> Artificial
<220>
   <223> PCR product
<400> 192
   gatgactatg gagacccaaa tgtctcagaa tgtatgtccc agaaacctgt ggctgcttca 60
   accattgaca gttttgctgc tgctggcttc tgcagacagt caagctgcag ctcccccaaa 120
   ggctgtgctg aaacttg 137
<210> 193
   <211> 110
   <212> DNA
   <213> Artificial
<220>
   <223> PCR product
<400> 193
   tttcttaaat ggcccacatc aggacagctg ttccttggcc atcccaaagc tattctgggg     60
   gcaacagagg tgaaactact gggccatgga cagccactta actggatttc                110
<210> 194
   <211> 147
   <212> DNA
   <213> Artificial
<220>
   <223> PCR product
<400> 194
   accacctcca ccatcccatc cggccagcca accaccattg ccagcatctc acccatcaca     60
   accaccagtc ccaagcctac ctcccagaaa cattaaacct ccgtttgacc taaaaagccc    120
   tgtcaatgaa gacaatcaag atggtgt                                        147
<210> 195
   <211> 135
   <212> DNA
   <213> Artificial
<220>
   <223> PCR product
<400> 195
   cataccataa caatgacgac aacttcagtc agcagctggc cttactcctc ccacagaatg     60
   cgctttataa ccaatcatag cgaccaaccg ccacaaaact tctcagcaac accaaatgtt    120
   actacctgtc ccatg                                                     135
<210> 196
   <211> 144
   <212> DNA
   <213> Artificial
<220>
   <223> PCR product
<400> 196
   ctttgggtga gttgaacttc ttccattata gaaagaattg aaggctgaga aactcagcct     60
   ctatcatgtg gaacagctct gacgccaact tctcctgcta ccatgagtct gtgctgggct    120
   atcgttatgt tgcagttagc tggg                                           144
<210> 197
   <211> 84
   <212> DNA
   <213> Artificial
<220>
   <223> PCR product
<400> 197
   cccttcctac tcccagacac ccaccctcgc ttcagccaca gtttcctcat ctgtccagtg      60
   ggtaggttgg actggaaaat ctct                                              84
<210> 198
   <211> 98
   <212> DNA
   <213> Artificial
<220>
   <223> PCR product
<400> 198
   gatccatctg ggtcacatcc tcttcctgct tttgctccca gtggctgcag ctcagacgac      60
   tccaggagag agatcatcac tccctgcctt ttaccctg                              98
<210> 199
   <211> 114
   <212> DNA
   <213> Artificial
<220>
   <223> PCR product
<400> 199
   gccatgaatt tgacctctat gaaaacaaag actacattag aaactgcatc attggtaaag      60
   gacgcagcta caagggaaca gtatctatca ctaagagtgg catcaaatgt cagc           114
<210> 200
   <211> 79
   <212> DNA
   <213> Artificial
<220>
   <223> PCR product
<400> 200
   cagttcagca tcagttccaa gacacgtttc caggtcccta tgctgtgctc acgaaggaca 60
   ccatgccgca gacgtacaa 79
<210> 201
   <211> 110
   <212> DNA
   <213> Artificial
<220>
   <223> PCR product
<400> 201
   aaactgttcc gcagccagag cgctgatgcc cccggcccca cagagcgcga gcggctaaag 60
   aagatgttgt ctgagggctc cgtgggcgag gtacagtggg aggccgagtt 110
<210> 202
   <211> 104
   <212> DNA
   <213> Artificial
<220>
   <223> PCR product
<400> 202
   cctagaggat cttgttactg aataccacgg gaacttttcg gcctggagtg gtgtgtctaa 60
   gggactggct gagagtctgc agccagacta cagtgaacga ctct 104
<210> 203
   <211> 104
   <212> DNA
   <213> Artificial
<220>
   <223> PCR product
<400> 203
   cccgcatccc tcacatgaaa gaccccatcg gtgacagctt ccaaaacgac aagctggtgg 60
   tctgggaggc gggcaaagcc ggcctggagg agtgtctggt gact 104
<210> 204
   <211> 150
   <212> DNA
   <213> Artificial
<220>
   <223> PCR product
<400> 204
   tcctgctggt catctggaag gctctgatcc acctgagcga cctccgggag tacaggcgct      60
   ttgagaagga gaagctcaag tcccagtgga acaatgataa tccccttttc aagagcgcca     120
   ccacgacggt catgaacccc aagtttgctg                                      150
<210> 205
   <211> 127
   <212> DNA
   <213> Artificial
<220>
   <223> PCR product
<400> 205
   gcgggaggct gtctttccct tccagcctgg aagtgttgca gaggtgtgca tcaccttcga      60
   ccaggccaac ctgaccgtca agctgccaga tggatacgaa ttcaagttcc ccaaccgcct     120
   caacctg                                                               127
<210> 206
   <211> 150
   <212> DNA
   <213> Artificial
<220>
   <223> PCR product
<400> 206
   tgaagcccaa gagccaaagg aatcaccacc accttctaaa acgtcagcag ctgctcagtt      60
   ggatgagctc atggctcacc tgactgagat gcaggccaag gttgcagtga gagcagatgc     120
   tggcaagaag cacttaccag acaagcagga                                      150
<210> 207
   <211> 85
   <212> DNA
   <213> Artificial
<220>
   <223> PCR product
<400> 207
   ccttgacctt ggggagaacc agttggagac cttgccacct gacctcctga ggggtccgct      60
   gcaattagaa cggctacatc tagaa                                            85
<210> 208
   <211> 118
   <212> DNA
   <213> Artificial
<220>
   <223> PCR product
<400> 208
   atgtctcaag gctcatctgt tttgaatttc tcctatccca tctgtgaggc ggctctgccc     60
   aagttttctt tctgtggaag aaggaaagga gagcagattt actatgctgg gcctgtca      118
<210> 209
   <211> 116
   <212> DNA
   <213> Artificial
<220>
   <223> PCR product
<400> 209
   atattcatgt tttacaagga gaaacccatt gattggctcc tggaccatat tctctgggtg     60
   aaagtctgca accctgaaaa agatgcaaaa cattgtgata gacagaaagc aaatct        116
<210> 210
   <211> 133
   <212> DNA
   <213> Artificial
<220>
   <223> PCR product
<400> 210
   agagcgtgtc cccacagggc aacagcgttg accgcatggc agctccgaga gcagaggctc     60
   tatttgactt cactggaaac agcaaactgg agctgaattt caaagctgga gatgtgatct    120
   tcctcctcag tcg                                                       133
<210> 211
   <211> 87
   <212> DNA
   <213> Artificial
<220>
   <223> PCR product
<400> 211
   ggggagcact actccgagaa gttacgagaa ctggtcagca tgtgcatctg ccctgacccc 60
   caccagagac ctgacatcgg atacgtg 87
<210> 212
   <211> 125
   <212> DNA
   <213> Artificial
<220>
   <223> PCR product
<400> 212
   agaggttata atctgaatcc caaaggagac tgcagctgat gaaagtgctt ccaaactgaa 60
   aattggacgt gcctttacga tggtaagcgt taacagctcc cactgcttct ataatgactc 120
   cttta 125
<210> 213
   <211> 142
   <212> DNA
   <213> Artificial
<220>
   <223> PCR product
<400> 213
   gactacagca acgaggagca catgaccctg ctgaagatga ttttgataaa atgctgtgat 60
   atctctaacg aggtccgtcc aatggaagtc gcagagcctt gggtggactg tttattagag 120
   gaatatttta tgcagagcga cc 142
<210> 214
   <211> 81
   <212> DNA
   <213> Artificial
<220>
   <223> PCR product
<400> 214
   acatgtattc aactgcacca agacctcgtg ttgagacctc ccgcgcagtg cctctggctg 60
   gttttggtta tggattgccc a 81
<210> 215
   <211> 125
   <212> DNA
   <213> Artificial
<220>
   <223> PCR product
<400> 215
   gatcagactc agcctcctca gagcctgttg ggatatatca gggtttcgag aagaagaccg     60
   gagttgctgg ggaggacatg caagacaaca gtgggaccta cggcaagatc tgggagggca    120
   gcagc                                                                125
<210> 216
   <211> 105
   <212> DNA
   <213> Artificial
<220>
   <223> PCR product
<400> 216
   tggcccagag tggcatgtcc cagccagacc cctcctggcc gctactcttt ttccctcttc     60
   tctgggctct cccactcctg gggccggtct gagcctacct gagcc                    105
<210> 217
   <211> 96
   <212> DNA
   <213> Artificial
<220>
   <223> PCR product
<400> 217
   gggtttccag cagcatttgt tgcagcatgg gctgtggcac gagcaactct ggctgatgcg     60
   aggtgctggg aacttagtgc tggagacatc aagtgg                               96
<210> 218
   <211> 140
   <212> DNA
   <213> Artificial
<220>
   <223> PCR product
<400> 218
   gccttctacc tgaagcagtg gcgctcctac aacatctcca tctgggacac cgcagggcgg     60
   gagcagttcc acggcctggg ctccatgtac tgccgggggg cggccgccat catcctcacc    120
   tatgatgtga atcaccggca                                                140
<210> 219
   <211> 140
   <212> DNA
   <213> Artificial
<220>
   <223> PCR product
<400> 219
   gccaacatca atgtggaaaa tgcatttttc actctcgcca gagatatcaa agcaaaaatg      60
   gacaaaaaat tggaaggcaa cagcccccag gggagcaacc agggagtcaa aatcacaccg     120
   gaccagcaga agaggagcag                                                 140
<210> 220
   <211> 119
   <212> DNA
   <213> Artificial
<220>
   <223> PCR product
<400> 220
   agggaccgct ctcttctctc gccgacagct tttccttccc tccatgagaa agaagccagc      60
   tctgtctgac ggcagcaatc ctgacggcga tctcctccag gaacactggc tggttgagg      119
<210> 221
   <211> 91
   <212> DNA
   <213> Artificial
<220>
   <223> PCR product
<400> 221
   ccctgaaccc cagaacaacc agctggatca gttctcacag gagctacagc gcggagactg      60
   ggaaacatgg ttccaaaact gttcacttcc c                                     91
<210> 222
   <211> 148
   <212> DNA
   <213> Artificial
<220>
   <223> PCR product
<400> 222
   aagctctttg ggctggtgat ggccttgtcg gctgtggtgt ctctgctcca gttccccatc 60
   ttcaccctca tcaaaggctc ccttcagaat gacccatttt acgtgaatgt gatgttcatg 120
   cttgccattc ttctgacatt cttccacc 148
<210> 223
   <211> 150
   <212> DNA
   <213> Artificial
<220>
   <223> PCR product
<400> 223
   tacgacatgc tggggatcat ggcatggaat gcaacttgca aaaactggct ggcagcagag 60
   gctgccctgg aaaagtacta cctttccatt ttttatggga ttgagttcgt tgtgggagtc 120
   cttggaaata ccattgttgt ttacggctac 150
<210> 224
   <211> 112
   <212> DNA
   <213> Artificial
<220>
   <223> PCR product
<400> 224
   actgttggct gtgaggaatg cacagtgttt ccctgtttat ccatcccctg caaactgcag 60
   agtggcactc attgcttgtg gacggaccag ctcctccaag gctctgaaaa gg 112
<210> 225
   <211> 140
   <212> DNA
   <213> Artificial
<220>
   <223> PCR product
<400> 225
   ccaggcagtc agatcatctt ctcgaacccc gagtgacaag cctgtagccc atgttgtagc 60
   aaaccctcaa gctgaggggc agctccagtg gctgaaccgc cgggccaatg ccctcctggc 120
   caatggcgtg gagctgagag 140
<210> 226
   <211> 78
   <212> DNA
   <213> Artificial
<220>
   <223> PCR product
<400> 226
   cctgcaagcc ctggaccaac tgcaccttgg ctgggaagca caccctgcag ccggccagca      60
   atagctcgga cgcaatct                                                    78
<210> 227
   <211> 97
   <212> DNA
   <213> Artificial
<220>
   <223> PCR product
<400> 227
   accagctcca ggagaaggca actccagtca gaacagcaga aataagcgtg ccgttcaggg      60
   tccagaagaa acagtcactc aagactgctt gcaactg                               97
<210> 228
   <211> 100
   <212> DNA
   <213> Artificial
<220>
   <223> PCR product
<400> 228
   tccgagaagt ggttgctgac agccacaaag tgaaagggag tgaggcggcg tggacgagta      60
   aggagtgaca gtgaggattc acatttgggt tatttcaaga                           100
<210> 229
   <211> 80
   <212> DNA
   <213> Artificial
<220>
   <223> PCR product
<400> 229
   ctgagaccga gtcgccttat caggagctcc agggtcagag gtcggatgtc tacagcgacc      60
   tcaacacaca gaggccgtat                                                  80
<210> 230
   <211> 82
   <212> DNA
   <213> Artificial
<220>
   <223> PCR product
<400> 230
   agtgctgtga tgatggacaa ttacatagta ccgataacag ccatgcactg tgcaaagcat     60
   gcccttctgc acaggagagc aa                                               82
<210> 231
   <211> 149
   <212> DNA
   <213> Artificial
<220>
   <223> PCR product
<400> 231
   gtcggcatct gagaccagtg agaaacgccc cttcatgtgt gcttacccag gctgcaataa     60
   gagatatttt aagctgtccc acttacagat gcacagcagg aagcacactg gtgagaaacc    120
   ataccagtgt gacttcaagg actgtgaac                                       149
<210> 232
   <211> 124
   <212> DNA
   <213> Artificial
<220>
   <223> PCR product
<400> 232
   cccagaggaa acggacacaa ctccgggaag cctttgagca gctccaggcc aagaaacaaa     60
   tggccatgga gaaacgcaga gcagtccaga accagtggca gctacaacag gagaagcatc    120
   tgca                                                                  124